# EUROPEAN PATENT SPECIFICATION

(11) **EP 4 455 141 B1**
(45) Date of publication and mention of the grant of the patent: **25.03.2026**
(21) Application number: 22910193.6
(22) Date of filing: 23.12.2022
(51) Int. Cl.: C07D 405/14, C07C 59/245, C07C 59/255, C07C 215/08, C07C 215/10, C07C 279/14, C07C 309/30, A61P 3/10, A61K 31/454

(54) **PHARMACEUTICALLY ACCEPTABLE SALT AND CRYSTALLINE FORM OF A GLP-1 RECEPTOR AGONIST FOR THE TREATMENT OF DIABETES**
PHARMAZEUTISCH AKZEPTABLES SALZ UND KRISTALLINE FORM EINES GLP-1-REZEPTORAGONISTEN ZUR BEHANDLUNG VON DIABETES
SEL PHARMACEUTIQUEMENT ACCEPTABLE ET FORME CRISTALLINE D'UN AGONISTE DU RÉCEPTEUR GLP-1 POUR LE TRAITEMENT DU DIABÈTE

(30) Priority: 23.12.2021 CN 202111584574
(43) Date of publication of application: 30.10.2024
(60) Divisional application: 26158421.3
(73) Proprietor: Jiangsu Hengrui Pharmaceuticals Co., Ltd., Lianyungang, Jiangsu 222047 (CN)
(72) Inventor: LU, Weidong, Shanghai 200245 (CN); XU, Gujun, Lianyungang, Jiangsu 222047 (CN); YANG, Junran, Lianyungang, Jiangsu 222047 (CN); SHAO, Qiyun, Shanghai 200245 (CN); DU, Zhenxing, Lianyungang, Jiangsu 222047 (CN); FENG, Jun, Shanghai 200245 (CN); HE, Feng, Shanghai 200245 (CN)
(74) Representative: J A Kemp LLP
(86) International application number: PCT/CN2022/141418
(87) International publication number: WO 2023/116882

(56) References cited:
- EP-A1- 4 209 490
- WO-A1-2019/239319
- WO-A1-2021/249492
- WO-A1-2022/007979
- WO-A1-2022/228490
- CN-A- 112 533 674
- CN-A- 113 227 068

## Description

### TECHNICAL FIELD

The present disclosure belongs to the field of medicines, and relates to a pharmaceutically acceptable salt and a crystalline form of a GLP-1 receptor agonist and a preparation method therefor.

### BACKGROUND

Glucagon-like peptide-1 (GLP-1) is an incretin hormone secreted by L-cells in the lower digestive tract. GLP-1 plays a corresponding role by binding to its ubiquitous specific receptor. Organs in which GLP-1 receptor is now clearly present include islet cells, gastrointestinal, pulmonary, brain, kidney, hypothalamus and cardiovascular systems, and GLP-1 receptor may also be present in liver, adipose tissues and skeletal muscle. GLP-1 not only acts on β cells to promote insulin secretion, but also acts on α cells to inhibit glucagon secretion. There is generally no significant difference in serum GLP-1 levels in patients with normal glucose tolerance, impaired glucose tolerance, and type II diabetes. However, there is a deficiency of the response of β cells to GLP-1 after eating, and under certain conditions, the response is significantly enhanced after continuous infusion of GLP-1. Since the duration of action of human GLP-1 is very short (t1/2 < 1.5 minutes via intravenous injection), human GLP-1 is not suitable for clinical treatment of diabetes.

Peptidic GLP-1 receptor agonists (e.g., liraglutide and exenatide) have effects on improving blood glucose level in type II diabetic patients by lowering fasting and postprandial glucose. However, since the peptidic GLP-1 has poor oral bioavailability and is inconvenient to take, small molecule agonists of GLP-1 receptors with good oral bioavailability are highly desirable.

2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid is a novel GLP-1 receptor agonist (the structural formula is as follows, WO 2022/007979),

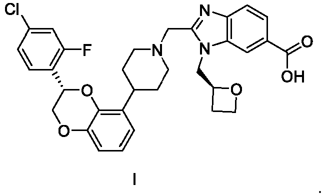

Half of the new molecular entities approved by the US Food and Drug Administration (FDA) are pharmaceutical salts. Since the first salt form was approved in 1939, salt drugs have been on a growing trajectory. In addition, salt formation can improve some undesirable physical, chemical or biological properties of drugs. It is of great significance to develop salts that are more excellent in physical and chemical properties or pharmaceutical properties than 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In addition, in view of the importance of solid drug crystal forms and their stability in clinical treatment, it is also of great significance to study the polymorphism of a pharmaceutically acceptable salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid in depth for developing drugs suitable for industrial production and with good biological activity.

WO 2019/239319 describes 6-carboxylic acids of benzimidazoles and 4-aza- 5-aza-, and 7-aza-benzimidazoles as GLP-1 R agonists, processes to make said compounds, and methods comprising administering said compounds to a mammal in need thereof.

### CONTENT OF THE PRESENT INVENTION

The present disclosure provides a pharmaceutically acceptable salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the pharmaceutically acceptable salt is selected from a tromethamine salt, an ammonium salt, a potassium salt, an arginine salt, a sodium salt, a meglumine salt, an ethanolamine salt, a p-toluenesulfonate salt, a tartrate salt, a sulfate salt, a malate salt, and a hydrochloride salt.

In some other embodiments, the chemical ratio of the compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid to an alkali molecule (a cation) is 1 : 0.5 to 1 : 3, including 1 : 0.5, 1 : 1, 1 : 2, or 1 : 3.

In some embodiments, the chemical ratio of the compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid to an alkali molecule is 1 : 1 or 1 : 2.

In some other embodiments, the chemical ratio of the compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid to an acid molecule (an acid radical) is 1 : 0.5 to 1 : 3, including 1 : 0.5, 1 : 1, 1 : 2, or 1 : 3.

In some embodiments, the chemical ratio of the compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid to an acid molecule is 1 : 1 or 1 : 2.

In another aspect, the present disclosure further provides a method for preparing the above-mentioned pharmaceutically acceptable salt, the method comprising a step of salt formation between compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid and an acid, or a step of salt formation between compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid and an alkali.

In some embodiments, a solvent used in the salt formation reaction is selected from at least one of methanol, 2-butanone, ethyl acetate, 1,4-dioxane, methyl isobutyl ketone, methyl tert-butyl ether, dichloromethane, ethanol, isopropanol, tetrahydrofuran, dimethyl sulfoxide, acetone, acetonitrile, toluene, isopropyl acetate, and water.

In some other embodiments, the volume (µl) of the solvent used in the salt formation reaction may be 1-200 times the mass (mg) of the above-mentioned compound, and in nonlimiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200. In some embodiments, the preparation method described in the present disclosure further comprises the step of centrifugation, washing, or drying.

In one aspect, the present disclosure provides an amorphous form of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, and an X-ray powder diffraction pattern expressed with diffraction angle 2*θ* has no obvious characteristic peaks.

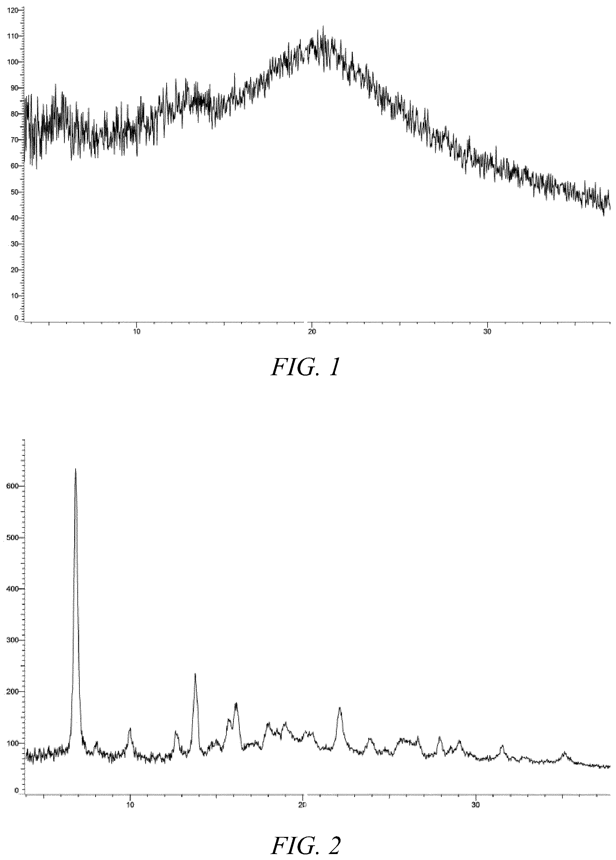

In some embodiments, the amorphous form of the tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid has an X-ray powder diffraction pattern expressed with diffraction angle 2*θ* as shown in FIG. 1.

In another aspect, the present disclosure further provides crystal form A of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form A of the tromethamine salt has an X-ray powder diffraction pattern expressed with diffraction angle 2*θ* in which there are characteristic peaks at 6.850, 13.789, 16.148, and 22.138.

In some embodiments, the crystal form A of the tromethamine salt has an X-ray powder diffraction pattern expressed with diffraction angle 2*θ* in which there are characteristic peaks at 6.850, 9.982, 13.789, 16.148, and 22.138.

In some embodiments, the crystal form A of the tromethamine salt has an X-ray powder diffraction pattern expressed with diffraction angle 2*θ* in which there are characteristic peaks at 6.850, 9.982, 12.697, 13.789, 15.763, 16.148, 18.016, 19.016, and 22.138.

In some embodiments, the crystal form A of the tromethamine salt has an X-ray powder diffraction pattern expressed with diffraction angle 2*θ* in which there are characteristic peaks at 6.850, 8.051, 9.982, 12.697, 13.789, 14.704, 14.990, 15.763, 16.148, 18.016, 19.016, 20.109, 22.138, 25.712, and 27.909.

In some embodiments, the crystal form A of the tromethamine salt has an X-ray powder diffraction pattern expressed with diffraction angle 2*θ* as shown in FIG. 2.

In another aspect, the present disclosure further provides crystal form B of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form B of the tromethamine salt has an X-ray powder diffraction pattern expressed with diffraction angle 2*θ* in which there are characteristic peaks at 8.144, 10.511, 12.290, 20.632, and 21.699.

In some embodiments, the crystal form B of the tromethamine salt has an X-ray powder diffraction pattern expressed with diffraction angle 2*θ* in which there are characteristic peaks at 8.144, 10.511, 12.290, 13.996, 14.665, 20.632, 21.699, and 24.74.

In some embodiments, the crystal form B of the tromethamine salt has an X-ray powder diffraction pattern expressed with diffraction angle 2*θ* in which there are characteristic peaks at 8.144, 10.511, 12.290, 13.996, 14.665, 15.607, 16.520, 18.883, 20.632, 21.699, 23.741, 24.743, 26.055, and 27.035.

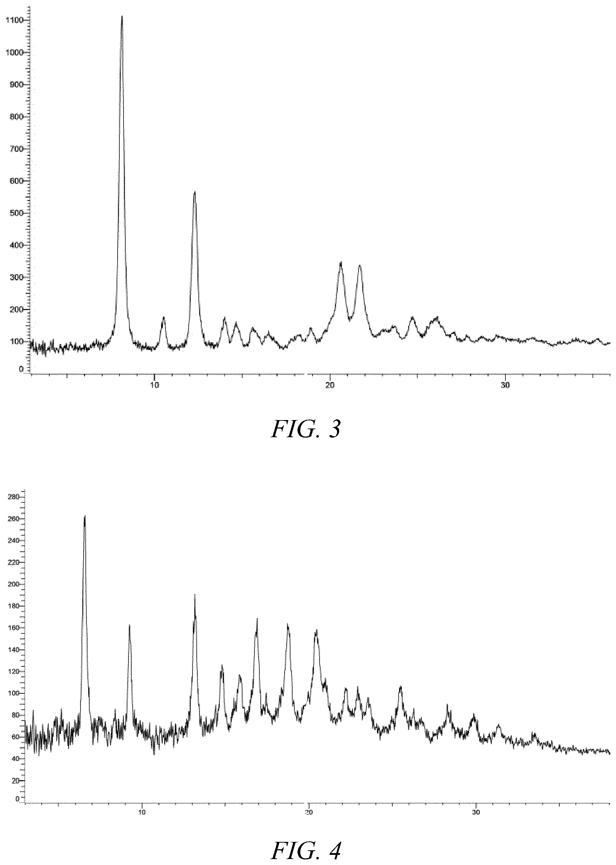

In some embodiments, the crystal form B of the tromethamine salt has an X-ray powder diffraction pattern expressed with diffraction angle 2*θ* as shown in FIG. 3.

In another aspect, the present disclosure further provides crystal form C of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form C of the tromethamine salt has an X-ray powder diffraction pattern expressed with diffraction angle 2*θ* in which there are characteristic peaks at 6.551, 9.269, 13.175, and 16.906.

In some embodiments, the crystal form C of the tromethamine salt has an X-ray powder diffraction pattern expressed with diffraction angle 2*θ* in which there are characteristic peaks at 6.551, 9.269, 13.175, 14.802, 15.864, 16.906, 18.776, 20.430, and 25.496.

In some embodiments, the crystal form C of the tromethamine salt has an X-ray powder diffraction pattern expressed with diffraction angle 2*θ* in which there are characteristic peaks at 6.551, 8.392, 9.269, 13.175, 14.802, 15.864, 16.906, 18.776, 20.430, 22.211, 22.922, 23.574, 25.496, and 26.290.

In some embodiments, the crystal form C of the tromethamine salt has an X-ray powder diffraction pattern expressed with diffraction angle 2*θ* as shown in FIG. 4.

In another aspect, the present disclosure further provides crystal form D of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form D of the tromethamine salt has an X-ray powder diffraction pattern expressed with diffraction angle 2*θ* in which there are characteristic peaks at 5.281, 10.292, 13.322, and 21.390.

In some embodiments, the crystal form D of the tromethamine salt has an X-ray powder diffraction pattern expressed with diffraction angle 2*θ* in which there are characteristic peaks at 5.281, 9.912, 10.292, 10.961, 13.322, 21.390, 22.215, 23.979, 25.029, and 25.846.

In some embodiments, the crystal form D of the tromethamine salt has an X-ray powder diffraction pattern expressed with diffraction angle 2*θ* in which there are characteristic peaks at 5.281, 9.912, 10.292, 10.961, 11.613, 13.322, 15.351, 18.283, 19.214, 21.390, 22.215, 23.471, 23.979, 25.029, 25.846, 27.918, and 30.121.

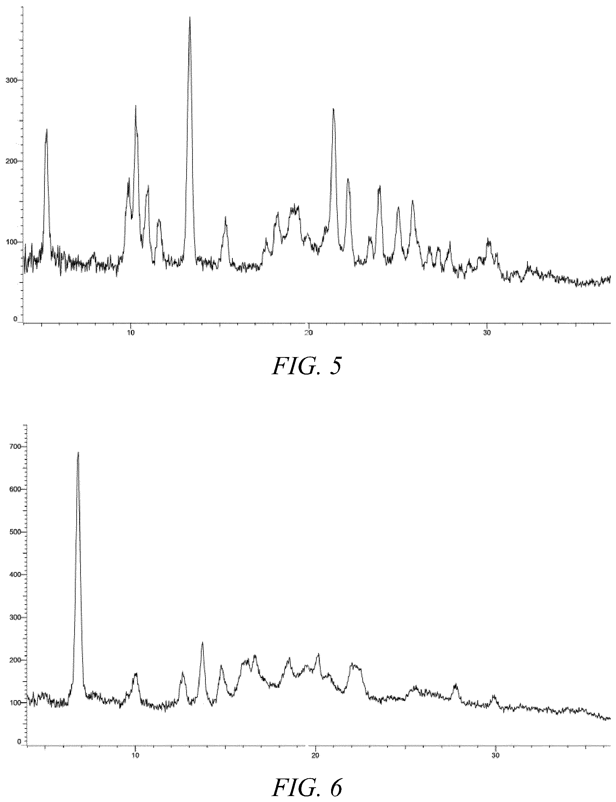

In some embodiments, the crystal form D of the tromethamine salt has an X-ray powder diffraction pattern expressed with diffraction angle 2*θ* as shown in FIG. 5.

In another aspect, the present disclosure further provides crystal form E of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form E of the tromethamine salt has an X-ray powder diffraction pattern expressed with diffraction angle 2*θ* in which there are characteristic peaks at 6.821, 10.035, 12.653, 13.727, and 14.787.

In some embodiments, the crystal form E of the tromethamine salt has an X-ray powder diffraction pattern expressed with diffraction angle 2*θ* in which there are characteristic peaks at 6.821, 10.035, 12.653, 13.727, 14.787, 16.081, 16.648, 18.571, 20.151, and 22.204.

In some embodiments, the crystal form E of the tromethamine salt has an X-ray powder diffraction pattern expressed with diffraction angle 2*θ* as shown in FIG. 6.

In another aspect, the present disclosure further provides a method for preparing the above-mentioned crystal form A, B, C, D, or E, the method comprising (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, tromethamine and a solvent, and dissolving the mixture by means of stirring or heating, and (b) crystallization.

In some embodiments, the method for preparing the above-mentioned crystal form A comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, tromethamine, and a solvent (1), dissolving the mixture by means of stirring or heating, and (b) crystallization, wherein the solvent (1) is selected from one or more of ethanol, ethyl acetate, methyl tert-butyl ether or tetrahydrofuran, isopropanol, methanol, and water.

In some embodiments, the method for preparing the above-mentioned crystal form B comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, tromethamine, and 10% water/acetone solvent, and dissolving the mixture by means of stirring or heating, and (b) crystallization.

In some embodiments, the method for preparing the above-mentioned crystal form C comprises (a) mixing crystal form A of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with one or more solvents of ethanol, acetone, isopropyl acetate, dioxane, and toluene, and (b) crystallizing.

In some embodiments, the method for preparing the above-mentioned crystal form D comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, tromethamine and one or more solvents of methanol and isopropanol, dissolving the mixture by means of stirring or heating, and (b) crystallization.

In some embodiments, the method for preparing the above-mentioned crystal form E comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, tromethamine, and acetonitrile solvent, and dissolving the mixture by means of stirring or heating, and (b) crystallization.

In another aspect, the present disclosure further provides crystal form F of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form F of the tromethamine salt has an X-ray powder diffraction pattern expressed with diffraction angle 2*θ* in which there are characteristic peaks at 7.674, 10.614, 16.400, and 18.645.

In some embodiments, the crystal form F of the tromethamine salt has an X-ray powder diffraction pattern expressed with diffraction angle 2*θ* in which there are characteristic peaks at 6.777, 7.674, 10.614, 11.594, 14.408, 14.882, 16.400, and 18.645.

In some embodiments, the crystal form F of the tromethamine salt has an X-ray powder diffraction pattern expressed with diffraction angle 2*θ* in which there are characteristic peaks at 6.777, 7.674, 10.614, 11.594, 13.671, 14.408, 14.882, 16.400, 18.645, 20.849, 21.384, 21.731, 22.108, 24.721, 26.169, and 29.192.

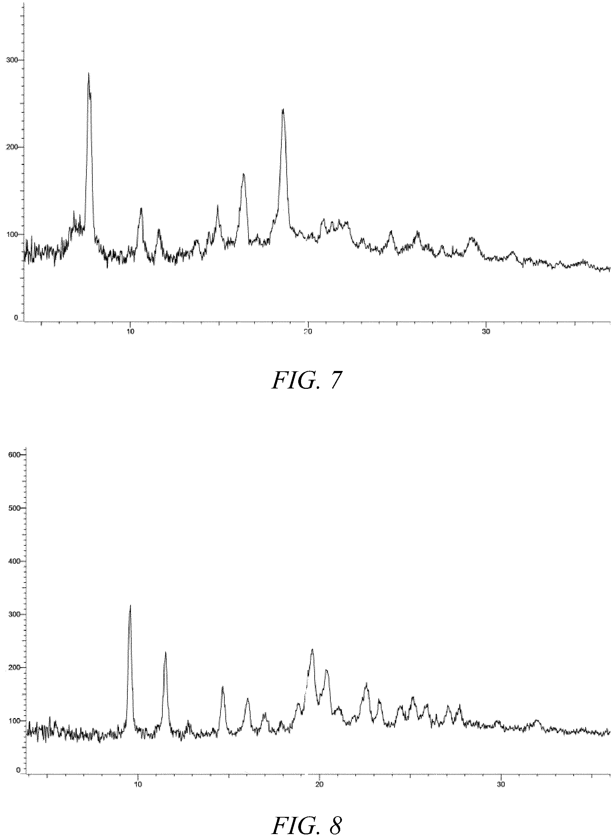

In some embodiments, the crystal form F of the tromethamine salt has an X-ray powder diffraction pattern expressed with diffraction angle 2*θ* as shown in FIG. 7.

In another aspect, the present disclosure further provides a method for preparing the crystal form F of the tromethamine salt. In some embodiments, the method for preparing the crystal form F of the tromethamine salt comprises a step of (a) placing crystal form A of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid at 80 °C/0% RH for 96 h or drying same at 167 °C.

In another aspect, the present disclosure further provides crystal form α of a potassium salt of compound 2-((4-((*S*)-3-(4-chloro-2-fluorophenyl)-2,3-dihydrobenzo[b][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form α of the potassium salt has an X-ray powder diffraction pattern expressed with diffraction angle 2*θ* in which there are characteristic peaks at 9.564, 11.515, 14.683, 19.607, and 20.391.

In some embodiments, the crystal form α of the potassium salt has an X-ray powder diffraction pattern expressed with diffraction angle 2*θ* in which there are characteristic peaks at 9.564, 11.515, 14.683, 16.058, 18.859, 19.607, 20.391, 22.592, 23.320, and 25.176.

In some embodiments, the crystal form α of the potassium salt has an X-ray powder diffraction pattern expressed with diffraction angle 2*θ* in which there are characteristic peaks at 9.564, 11.515, 14.683, 16.058, 17.033, 18.859, 19.607, 20.391, 21.064, 22.592, 23.320, 24.449, 25.176, 25.933, 27.080, and 27.708.

In some embodiments, the crystal form α of the potassium salt has an X-ray powder diffraction pattern expressed with diffraction angle 2*θ* as shown in FIG. 8.

The present disclosure further provides a method for preparing the crystal form α of the potassium salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with a solvent (2), and adding a potassium hydroxide solution, and (b) pulping, wherein the solvent (2) is selected from one or more of ethyl acetate or methyl tert-butyl ether.

In another aspect, the present disclosure further provides crystal form I of a sodium salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form I of the sodium salt has an X-ray powder diffraction pattern expressed with diffraction angle 2*θ* in which there are characteristic peaks at 5.257, 5.806, 6.795, 10.106, 12.203, and 20.693.

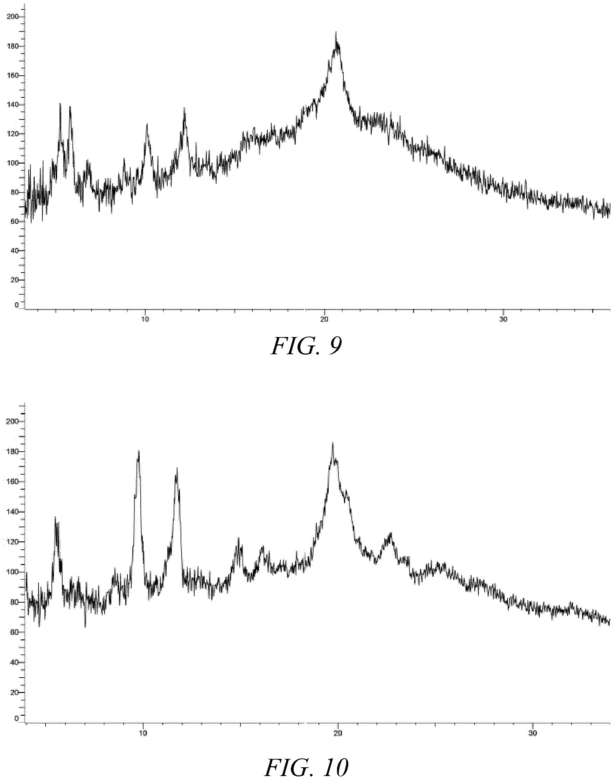

In some embodiments, the crystal form I of the sodium salt has an X-ray powder diffraction pattern expressed with diffraction angle 2*θ* as shown in FIG. 9.

The present disclosure further provides a method for preparing the crystal form I of the sodium salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with a solvent (3), and adding a sodium hydroxide solution, and (b) pulping, wherein the solvent (3) is selected from one or more of methanol or ethyl acetate.

In another aspect, the present disclosure further provides crystal form II of a sodium salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form II of the sodium salt has an X-ray powder diffraction pattern expressed with diffraction angle 2*θ* in which there are characteristic peaks at 9.754, 11.731, and 19.730.

In some embodiments, the crystal form II of the sodium salt has an X-ray powder diffraction pattern expressed with diffraction angle 2*θ* in which there are characteristic peaks at 5.574, 9.754, 11.731, 14.856, 16.091, 19.730, and 22.670.

In some embodiments, the crystal form II of the sodium salt has an X-ray powder diffraction pattern expressed with diffraction angle 2*θ* as shown in FIG. 10.

The present disclosure further provides a method for preparing the crystal form II of the sodium salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with a solvent (4), and adding a sodium hydroxide solution, and (b) crystallization, wherein the solvent (4) is selected from one or more of acetone or acetonitrile.

In another aspect, the present disclosure further provides crystal form I of a meglumine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form I of the meglumine salt has an X-ray powder diffraction pattern expressed with diffraction angle 2*θ* in which there are characteristic peaks at 5.498, 11.013, 14.626, and 17.942.

In some embodiments, the crystal form I of the meglumine salt has an X-ray powder diffraction pattern expressed with diffraction angle 2*θ* in which there are characteristic peaks at 5.498, 8.901, 11.013, 14.626, 17.942, 19.454, 22.668, and 25.696.

In some embodiments, the crystal form I of the meglumine salt has an X-ray powder diffraction pattern expressed with diffraction angle 2*θ* in which there are characteristic peaks at 5.498, 8.314, 8.901, 11.013, 11.891, 12.810, 14.626, 15.683, 15.931, 17.942, 18.748, 19.454, 20.393, 21.419, 22.234, 22.668, 23.391, 24.739, and 25.696.

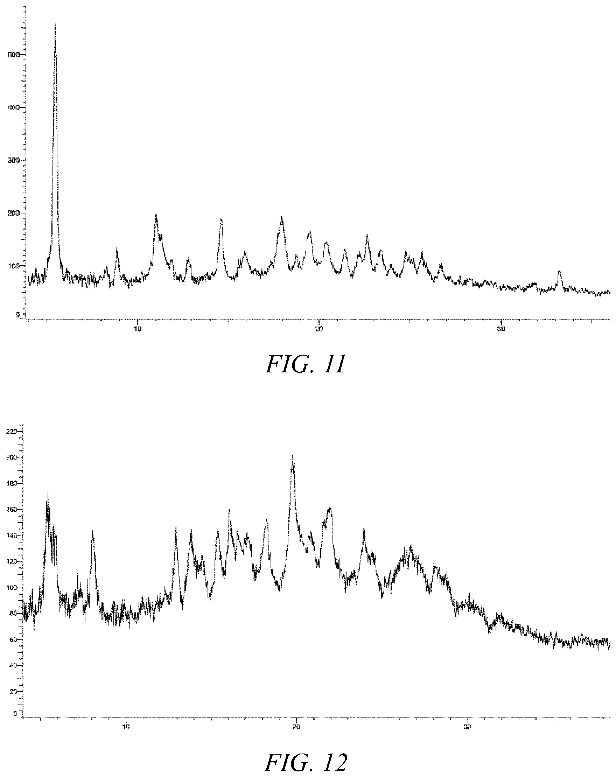

In some embodiments, the crystal form I of the meglumine salt has an X-ray powder diffraction pattern expressed with diffraction angle 2*θ* as shown in FIG. 11.

The present disclosure further provides a method for preparing the crystal form I of the meglumine salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with acetonitrile, and adding meglumine, and (b) crystallization.

In another aspect, the present disclosure further provides crystal form I of a p-toluenesulfonate salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form I of the p-toluenesulfonate salt has an X-ray powder diffraction pattern expressed with diffraction angle 2*θ* in which there are characteristic peaks at 5.453, 5.884, 8.063, 12.925, 16.071, and 19.778.

In some embodiments, the crystal form I of the p-toluenesulfonate salt has an X-ray powder diffraction pattern expressed with diffraction angle 2*θ* in which there are characteristic peaks at 5.453, 5.884, 8.063, 12.925, 13.825, 15.399, 16.071, 18.231, 19.778, and 21.917.

In some embodiments, the crystal form I of the p-toluenesulfonate salt has an X-ray powder diffraction pattern expressed with diffraction angle 2*θ* in which there are characteristic peaks at 5.453, 5.884, 8.063, 12.925, 13.825, 15.399, 16.071, 16.560, 17.066, 18.231, 19.778, 20.861, 21.917, 23.898, and 26.744.

In some embodiments, the crystal form I of the p-toluenesulfonate salt has an X-ray powder diffraction pattern expressed with diffraction angle 2*θ* as shown in FIG. 12.

The present disclosure further provides a method for preparing the crystal form I of the p-toluenesulfonate salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin -1-yl)methyl)-1-(((*S*)-oxetan-2-yl)methyl)-1*H*-benzo[*d*]imidazole-6-carboxylic acid with acetonitrile, and adding p-toluenesulfonic acid, and (b) crystallization.

In one aspect, the present disclosure provides an amorphous form of a tartrate salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, and an X-ray powder diffraction pattern expressed with diffraction angle 2*θ* has no obvious characteristic peaks.

In another aspect, the present disclosure further provides crystal form I of a tartrate salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form I of the tartrate salt has an X-ray powder diffraction pattern expressed with diffraction angle 2*θ* in which there are characteristic peaks at 12.810, 18.824, 21.890, and 24.472.

In some embodiments, the crystal form I of the tartrate salt has an X-ray powder diffraction pattern expressed with diffraction angle 2*θ* in which there are characteristic peaks at 12.810, 14.448, 15.604, 18.824, 20.410, 21.890, and 24.472.

In some embodiments, the crystal form I of the tartrate salt has an X-ray powder diffraction pattern expressed with diffraction angle 2*θ* in which there are characteristic peaks at 9.342, 10.132, 10.858, 11.272, 12.810, 14.448, 15.604, 18.824, 20.410, 21.890, 24.472, and 27.880.

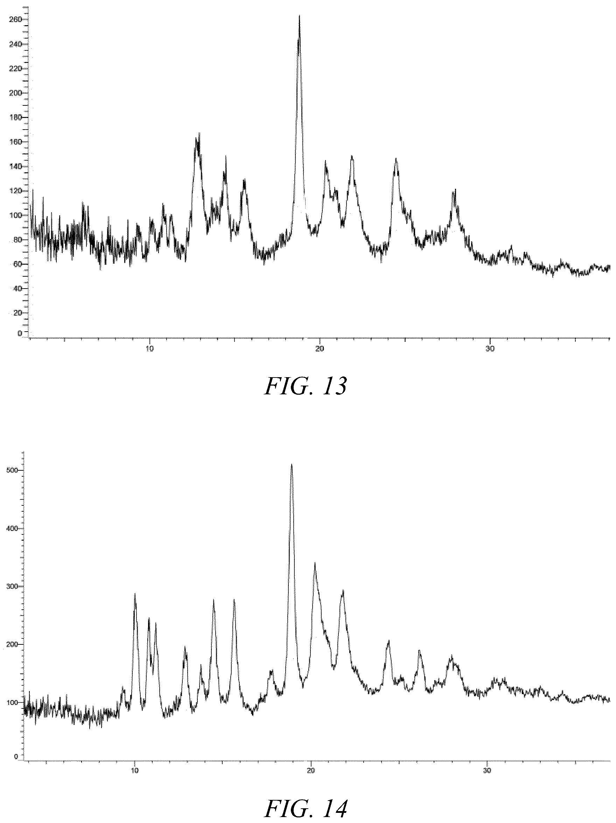

In some embodiments, the crystal form I of the tartrate salt has an X-ray powder diffraction pattern expressed with diffraction angle 2*θ* as shown in FIG. 13.

The present disclosure further provides a method for preparing the crystal form I of the tartrate salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with a solvent (5), and adding L-tartaric acid, and (b) crystallization, wherein the solvent (5) is selected from one or more of ethanol, ethyl acetate, and methyl tert-butyl ether.

In one aspect, the present disclosure provides an amorphous form of a malate salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, and an X-ray powder diffraction pattern expressed with diffraction angle 2*θ* has no obvious characteristic peaks.

In another aspect, the present disclosure further provides crystal form α of a malate salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form α of the malate salt has an X-ray powder diffraction pattern expressed with diffraction angle 2*θ* in which there are characteristic peaks at 10.050, 14.508, 15.693, 18.924, and 20.240.

In some embodiments, the crystal form α of the malate salt has an X-ray powder diffraction pattern expressed with diffraction angle 2*θ* in which there are characteristic peaks at 10.050, 10.818, 11.213, 12.871, 14.508, 15.693, 18.924, 20.240, 21.829, and 24.396.

In some embodiments, the crystal form α of the malate salt has an X-ray powder diffraction pattern expressed with diffraction angle 2*θ* in which there are characteristic peaks at 9.364, 10.050, 10.818, 11.213, 12.871, 13.774, 14.508, 15.693, 17.760, 18.924, 20.240, 21.829, 24.396, 26.200, 27.936, and 28.188.

In some embodiments, the crystal form α of the malate salt has an X-ray powder diffraction pattern expressed with diffraction angle 2*θ* as shown in FIG. 14.

The present disclosure further provides a method for preparing the crystal form α of the malate salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with a solvent (6), and adding malic acid, and (b) crystallization, wherein the solvent (6) is selected from one or more of ethyl acetate or methyl tert-butyl ether.

In one aspect, the present disclosure further provides an amorphous form of a hydrochloride salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, and an X-ray powder diffraction pattern expressed with diffraction angle 2*θ* has no obvious characteristic peaks.

In another aspect, the present disclosure further provides crystal form I of a hydrochloride salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid.

In some embodiments, the crystal form I of the hydrochloride salt has an X-ray powder diffraction pattern expressed with diffraction angle 2*θ* in which there are characteristic peaks at 9.939, 14.333, 14.933, 17.523, 18.480, and 20.134.

In some embodiments, the crystal form I of the hydrochloride salt has an X-ray powder diffraction pattern expressed with diffraction angle 2*θ* in which there are characteristic peaks at 9.939, 13.123, 13.649, 14.333, 14.933, 16.616, 17.523, 18.480, 19.378, 20.134, 20.988, 26.399, and 26.970.

In some embodiments, the crystal form I of the hydrochloride salt has an X-ray powder diffraction pattern expressed with diffraction angle 2*θ* in which there are characteristic peaks at 9.939, 12.267, 13.123, 13.649, 14.333, 14.933, 16.616, 17.523, 18.480, 19.378, 20.134, 20.988, 22.377, 23.002, 24.477, 25.322, 26.399, 26.970, 27.609, 30.822, and 33.760.

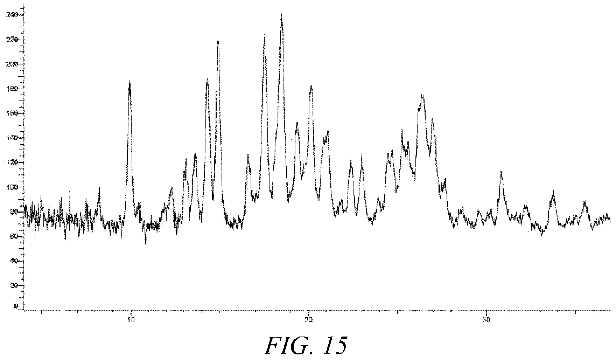

In some embodiments, the crystal form I of the hydrochloride salt has an X-ray powder diffraction pattern expressed with diffraction angle 2*θ* as shown in FIG. 15.

The present disclosure further provides a method for preparing the crystal form I of the hydrochloride salt. In some embodiments, the method comprises (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with water, and adding hydrochloric acid, and (b) crystallization.

The volume (µl) of the solvents (1), (2), (3), (4), (5), and (6) used in the present disclosure may be 1-200 times the mass (mg) of the above-mentioned compound, and in nonlimiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

In addition, the method for preparing the above-mentioned crystal forms in the present disclosure further comprises one or more steps of filtration, washing, or drying.

The present disclosure further provides a pharmaceutical composition, which contains the above-mentioned pharmaceutically acceptable salt or a crystal form thereof, and optionally pharmaceutical auxiliaries selected from pharmaceutically acceptable excipients.

The present disclosure further provides a method for preparing a pharmaceutical composition, the method comprising a step of mixing the above-mentioned pharmaceutically acceptable salt or a crystal form thereof with a pharmaceutically acceptable excipient.

The present disclosure further provides the use of the above-mentioned pharmaceutically acceptable salt or crystal form thereof or the above-mentioned composition in the preparation of a medicament for treating or preventing a disease associated with GLP-1 receptor.

The present disclosure further provides the use of the above-mentioned pharmaceutically acceptable salt or crystal form thereof or the above-mentioned composition in the preparation of a medicament for treating or preventing diabetes.

The "2*θ* or angle 2*θ*" mentioned in the present disclosure refers to a diffraction angle, and *θ* is a Bragg angle in ° or degrees; and the error range of 2*θ* for each characteristic peak is ± 0.2 (including the rounding of numbers with more than 1 decimal place), and may be -0.20, -0.19, -0.18, -0.17, -0.16, -0.15, -0.14, -0.13, -0.12, -0.11, -0.10, -0.09, -0.08, -0.07, -0.06, - 0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

According to the description of hygroscopic characteristics and the definition of hygroscopic weight gain in "9103 Guidelines for Hygroscopicity of Pharmaceuticals", Chinese Pharmacopoeia, Volume IV, 2015 edition, the following are defined:
deliquescent: absorbing enough water to form a liquid;
extremely hygroscopic: having a hygroscopic weight gain of not less than 15%;
hygroscopic: having a hygroscopic weight gain of less than 15% and not less than 2%;
slightly hygroscopic: having a hygroscopic weight gain of less than 2% and not less than 0.2%; and
no or almost no hygroscopicity: having a hygroscopic weight gain of less than 0.2%.

The "differential scanning calorimetry or DSC" mentioned in the present disclosure refers to measuring the temperature difference and heat flow difference between a sample and a reference during the process in which the sample is heated or maintained at a constant temperature, so as to characterize all physical and chemical changes related to thermal effects and obtain the phase change information of the sample.

In the present disclosure, the drying is generally at a temperature of 25 °C to 100 °C, preferably 40 °C to 70 °C, and may be either drying under atmospheric pressure or drying under reduced pressure with the pressure < -0.08 MPa.

The "excipient" mentioned in the present disclosure includes, but is not limited to, any adjuvant, carrier, glidant, sweetener, diluent, preservative, dye/colorant, flavoring agent, surfactant, wetting agent, dispersant, suspending agent, stabilizer, isotonic agent, or emulsifier that has been approved by the US Food and Drug Administration to be acceptable for human or livestock use.

The "pulping" mentioned in the present disclosure refers to a method of purification by means of the characteristic that a substance has poor solubility in a solvent while impurities have good solubility in the solvent. Pulping purification may result in discoloration, change the crystal form, or remove a small amount of impurities.

The starting raw materials used in the method for preparing the crystal forms in the present disclosure can be any form of compounds, and specific forms include, but are not limited to, amorphous form, any crystal form, hydrate, solvate, etc.

If the content of related substances is measured and calculated data, the numerical values in the present disclosure will inevitably have errors to some extent. Generally, ± 10% is within a reasonable error range. There is a certain degree of error variation in the context where it is used. The error variation does not exceed ± 10%, and may be ± 9%, ± 8%, ± 7%, ± 6%, ± 5%, ± 4%, ± 3%, ± 2%, or ± 1%, preferably ± 5%.

The compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid (hereinafter referred to as Compound A) in the present disclosure is prepared by referring to a method in WO2022/007979.

### BRIEF DESCRIPTION OF THE DRAWINGS

FIG. 1: an XRPD pattern of an amorphous form of a tromethamine salt.
FIG. 2: an XRPD pattern of crystal form A of a tromethamine salt.
FIG. 3: an XRPD pattern of crystal form B of a tromethamine salt.
FIG. 4: an XRPD pattern of crystal form C of a tromethamine salt.
FIG. 5: an XRPD pattern of crystal form D of a tromethamine salt.
FIG. 6: an XRPD pattern of crystal form E of a tromethamine salt.
FIG. 7: an XRPD pattern of crystal form F of a tromethamine salt.
FIG. 8: an XRPD pattern of crystal form α of a potassium salt.
FIG. 9: an XRPD pattern of crystal form I of a sodium salt.
FIG. 10: an XRPD pattern of crystal form II of a sodium salt.
FIG. 11: an XRPD pattern of crystal form I of a meglumine salt.
FIG. 12: an XRPD pattern of crystal form I of a p-toluenesulfonate salt.
FIG. 13: an XRPD pattern of crystal form I of a tartrate salt.
FIG. 14: an XRPD pattern of crystal form α of a malate salt.
FIG. 15: an XRPD pattern of crystal form I of a hydrochloride salt.

### DETAILED DESCRIPTION OF THE PREFERRED EMBODIMENT

The present disclosure will be explained in more detail below in conjunction with examples or experimental examples. The examples or experimental examples in the present disclosure are only used to illustrate the technical solutions of the present disclosure and do not limit the essence and scope of the present disclosure.

The reagents used in the present disclosure are commercially available.

Test conditions for instruments used in experiments in the present disclosure:
1. Differential Scanning Calorimeter (DSC)
   Instrument model: Mettler Toledo DSC 3+STARe System
   Purge gas: nitrogen; nitrogen purging speed: 50 mL/min
   Heating rate: 10.0 °C/min
   Temperature range: 25-350 °C or 25-300 °C
2. X-ray Powder Diffraction (XRPD)
   Instrument model: BRUKER D8 Discover X-ray powder diffractometer
   X-ray: monochromatic Cu-Kα ray (λ = 1.5406)
   Scanning mode: θ/2θ, scanning range (2θ range): 3° to 50°
   Voltage: 40 kV, current: 40 mA
3. Thermogravimetric Analysis (TGA)
   Instrument model: Mettler Toledo TGA2
   Purge gas: nitrogen; nitrogen purging speed: 50 mL/min
   Heating rate: 10.0 °C/min
   Temperature range: 30-350 °C
4. DVS, i.e., dynamic vapor sorption
   Surface Measurement Systems advantage 2 is used for detection at 25 °C. The humidity is 50%-95%-0%-95%-50% RH. The step is 10%. The criterion is that the mass change dM/dT for each step is less than 0.002%. TMAX is 360 min. Two cycles are carried out.
5. The progress of the reaction in the examples is monitored by means of thin layer chromatography (TLC). The developing solvent used in the reaction, the eluent system of column chromatography used in the purification of compounds and the developing solvent system of thin layer chromatography include: A: Dichloromethane/methanol system, B: n-hexane/ethyl acetate system. The silica gel plate for thin layer chromatography is Yantai Huanghai HSGF254 or Qingdao GF254 silica gel plate, the specification of the silica gel plate used for thin layer chromatography (TLC) is 0.15 mm to 0.2 mm, and the specification of the silica gel plate used for separating and purifying products by thin layer chromatography is 0.4 mm to 0.5 mm. For silica gel column chromatography, Yantai Huanghai silica gel 200-300 mesh silica gel is generally used as a carrier.
6. The structures of the compounds were determined by nuclear magnetic resonance (NMR) spectroscopy and/or mass spectrometry (MS). NMR shifts (δ) are given in a unit of 10-6 (ppm).
   NMR spectra were measured using a Bruker AVANCE-400 nuclear magnetic resonance instrument, with deuterated dimethyl sulfoxide (DMSO-d6), deuterated chloroform (CDCl3) and deuterated methanol (CD3OD) as solvents and tetramethylsilane (TMS) as an internal standard.
   MS determination was carried out by means of Agilent 1200/1290 DAD-6110/6120 Quadrupole MS liquid chromatography-mass spectrometer (manufacturer: Agilent, MS model: 6110/6120 Quadrupole MS); waters ACQuity UPLC-QD/SQD (manufacturer: waters, MS model: waters ACQuity Qda Detector/waters SQ Detector); and THERMO Ultimate 3000-Q Exactive (manufacturer: THERMO, MS model: THERMO Q Exactive).
7. Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.
8. HPLC determination was carried out by means of Agilent 1260DAD high-performance liquid chromatograph (ACE Excel C18 150 × 4.6 mm chromatographic column) and Thermo Dionex Ultimate 3000 high-pressure liquid chromatograph (Waters Xbridge C18 150 × 4.6 mm chromatographic column).

### Example 1: Preparation and Activity Test of Compound A

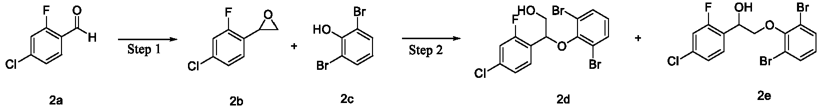

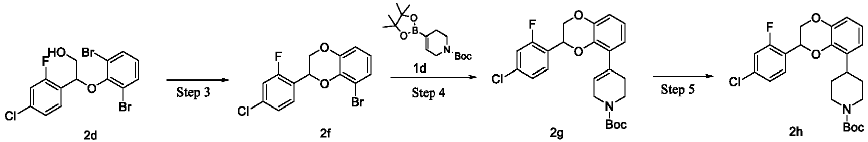

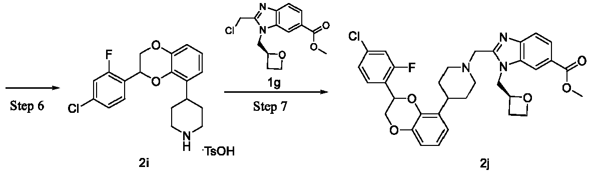

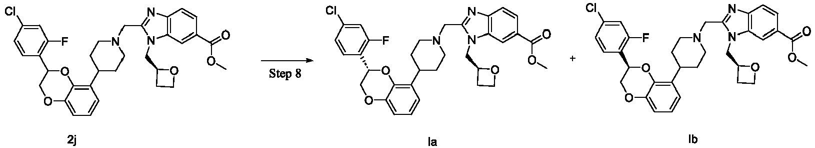

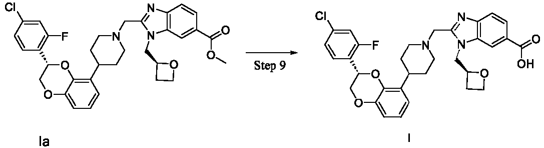

### Step 1

### 2-(4-Chloro-2-fluorophenyl)oxirane 2b

Potassium tert-butoxide (1.70 g, 15.14 mmol, Accela ChemBio (Shanghai) Inc.) was added to tetrahydrofuran (30 mL).Trimethylsulfonium iodide (3.09 g, 15.14 mmol, Adamas Reagent Co., Ltd.) was added under ice bath conditions. The mixture was stirred for 5 min. 4-Chloro-2-fluorobenzaldehyde **2a** (2.0 g, 12.61 mmol, Accela ChemBio (Shanghai) Inc.) was added. The mixture was filtered, diluted with ethyl acetate (80 mL), washed with saturated aqueous ammonium chloride solution (30 mL×2), washed with saturated brine (30 mL×2), dried over anhydrous sodium sulfate and concentrated under reduced pressure to remove the solvent. The resulting residue was purified by silica gel column chromatography with eluent system B to give the title compound **2b** (650 mg, yield: 29.9%).

¹ H NMR (500 MHz, CDCl₃) δ 7.05-7.13 (m, 3H), 4.01-4.15 (m, 1H), 3.17 (dd, 1H), 3.75 (dd, 1H).

### Step 2

### 2-(4-Chloro-2-fluorophenyl)-2-(2,6-dibromophenoxy)ethanol 2d

### 1-(4-Chloro-2-fluorophenyl)-2-(2,6-dibromophenoxy)ethanol 2e

After compound **2b** (520 mg, 3.01 mmol) and 2,6-dibromophenol **2c** (759 mg, 3.01 mmol, TCI (Shanghai) Co., Ltd.) were mixed, sodium methoxide (16 mg, 0.30 mmol, Adamas Reagent Co., Ltd.) was added. The mixture was stirred at 130 °C. for 2 h. After cooling, the resulting residue was purified by silica gel column chromatography with eluent system B to give the title compound **2d** (210 mg, yield: 16.4%) and compound **2e** (140 mg, yield: 10.9%).

**2d** MS m/z (ESI):422.9 [M-1].

**2d** ¹ H NMR (500 MHz, DMSO-d₆) δ 7.69 (t, 1H), 7.63 (d, 2H), 7.40 (dd, 1H), 7.35 (dd, 1H), 7.00 (t, 1H), 5.59 (t, 1H), 5.02 (t, 1H), 3.98-4.03 (m, 1H), 3.81-3.90 (m, 1H).

**2e** ¹ H NMR (500 MHz, DMSO-d₆) δ 7.64 (d, 2H), 7.62 (t, 1H), 7.39 (dd, 1H), 7.32 (dd, 1H), 7.02 (t, 1H), 5.82-6.01 (m, 1H), 5.28 (t, 1H), 4.07-4.12 (m, 1H), 3.95-4.00 (m, 1H).

### Step 3

### 8-Bromo-2-(4-chloro-2-fluorophenyl)-2,3-dihydrobenzo[b][1,4]dioxane 2f

Compound **2d** (595 mg, 1.40 mmol) was dissolved in anhydrous toluene (8 mL).S-1,1'-Bi-2-naphthol (159 mg, 0.55 mmol, Accela ChemBio (Shanghai) Inc.), cuprous iodide (52 mg, 0.27 mmol, Sinopharm Chemical Reagent Co., Ltd.) and cesium carbonate (912 mg, 2.80 mmol, Accela ChemBio (Shanghai) Inc.) were successively added. The mixture was heated at reflux and stirred for 18 h, cooled and concentrated under reduced pressure to remove the solvent. The resulting residue was purified by silica gel column chromatography with eluent system B to give the title compound **2f** (380 mg, yield: 78.9%).

MS m/z (ESI):343.1 [M-1].

¹ H NMR (500 MHz, DMSO-d₆) δ 7.57 (dd, 1H), 7.54 (t, 1H), 7.43 (dd, 1H), 7.19 (dd, 1H), 6.98 (dd, 1H), 6.85 (t, 1H), 5.58 (dd, 1H), 4.51 (dd, 1H), 4.20 (dd, 1H).

### Step 4

### tert-Butyl 4-(3-(4-chloro-2-fluorophenyl)-2,3-dihydrobenzo[b][1,4]dioxan-5-yl)-5,6-dihydropyridine-1(2H)-carboxylate 2g

Compound **2f** (354 mg, 1.03 mmol) and compound **1d** (350 mg, 1.13 mmol, Accela ChemBio (Shanghai) Inc.) were dissolved in 24 mL of a mixed solution of 1,4-dioxane and water (V/V=5 : 1). Sodium carbonate (218 mg, 2.06 mmol), tetrakis(triphenylphosphine)palladium (119 mg, 1.03 mmol) were added. The mixture was stirred at 90 °C. for 4 h under nitrogen, cooled to room temperature, filtered and concentrated under reduced pressure to remove the solvent. The resulting residue was purified by silica gel column chromatography with eluent system B to give the title compound **2g** (410 mg, yield: 89.2%).

MS m/z (ESI):390.1 [M-55].

¹ H NMR (500 MHz, CDCl₃) δ 7.39 (t, 1H), 7.19-7.23 (m, 1H), 7.15 (dd, 1H), 6.83-6.89 (m, 2H), 6.77-6.81 (m, 1H), 5.76-5.91 (m, 1H), 5.32-5.46 (m, 1H), 5.41 (dd, 1H), 3.99-4.08 (m, 2H), 3.97 (dd, 1H), 3.43-3.69 (m, 2H), 2.40-2.63 (m, 2H), 1.47 (s, 9H).

### Step 5

### tert-Butyl 4-(3-(4-chloro-2-fluorophenyl)-2,3-dihydrobenzo[b][1,4]dioxan-5-yl)piperidine -1-carboxylate 2h

Compound **2g** (220 mg, 0.49 mmol) was dissolved in ethyl acetate (10 mL) and 1,2-dichlorobenzene (0.5 mL, TCI (Shanghai) Co., Ltd.).10% palladium on carbon (50 mg, 0.47 mmol) was added. Hydrogenation was performed at room temperature for 1 h under one atmosphere of hydrogen. The mixture was filtered and concentrated under reduced pressure to remove the solvent. The resulting residue was purified by silica gel column chromatography with eluent system B to give the title compound **2h** (178 mg, yield: 80.5%).

MS m/z (ESI):392.1[M-55].

¹ H NMR (500 MHz, CDCl₃) δ 7.40 (t, 1H), 7.21-7.24 (m, 1H), 7.16 (dd, 1H), 6.82-6.88 (m, 1H), 6.76-6.81 (m, 2H), 5.35-5.45 (m, 1H), 4.40 (dd, 1H), 4.09-4.33 (m, 2H), 3.96 (dd, 1H), 2.99-3.11 (m, 1H), 2.67-2.90 (m, 2H), 1.72-1.91 (m, 2H), 1.58-1.69 (m, 2H), 1.46 (s, 9H).

### Step 6

### 4-(3-(4-Chloro-2-fluorophenyl)-2,3-dihydrobenzo[b][1,4]dioxan-5-yl)piperidine 4-methylbenzenesulfonate 2i

Compound **2h** (178 mg, 0.40 mmol) was dissolved in ethyl acetate (5 mL). p-Toluenesulfonic acid monohydrate (189 mg, 0.99 mmol) was added. The mixture was stirred at 60 °C. for 2 h. The mixture was cooled to room temperature and concentrated under reduced pressure to give the crude title product **2i** (206 mg).The product was directly used in the next step without being purified.

MS m/z (ESI):348.1 [M+1].

### Step 7

### Methyl 2-((4-(3-(4-chloro-2-fluorophenyl)-2,3-dihydrobenzo[b][1,4]dioxan-5-yl)piperidin -1-yl)methyl)-1-((S)-oxetan-2-ylmethyl)-1H-benzo[d]imidazole-6-carboxylate 2j

Compound **1g** (175 mg, 0.59 mmol) and **2i** (206 mg, 0.59 mmol) were dissolved in acetonitrile (10 mL). Potassium carbonate (410 mg, 2.97 mmol) was added. The mixture was stirred at 60 °C. for 3 h, filtered and concentrated under reduced pressure to remove the solvent. The resulting residue was purified by silica gel column chromatography with eluent system B to give the title mixture of diastereomers **2j** (207 mg, yield: 57.7%).

MS m/z (ESI):606.2 [M+1].

### Step 8

### Methyl 2-((4-((R)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylate Ia

### Methyl 2-((4-((R)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylate Ib

Compound **2j** (830 mg, 1.37 mmol) was resolved by preparative chiral chromatography (resolution conditions: CHIRALPAK IG 250×20 mm, 5 µm (with column protection); mobile phase: hexane/EtOH (0.1% DEA) = 70/30 (V/V), flow rate: 20 mL/min).The corresponding fractions were collected and concentrated under reduced pressure to give the title products (415 mg and 340 mg).

Single-configuration compound (shorter retention time **Ia**): MS m/z (ESI):606.0 [M+1]. Preparative chiral chromatography: retention time 13.653 min.

Single-configuration compound (longer retention time **Ib**): MS m/z (ESI):606.0 [M+1]. Preparative chiral chromatography: retention time 16.422 min.

### Step 9

### 2-((4-((S)-3-(4-Chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid I

**Ia** (415 mg, 0.68 mmol) was dissolved in 36 mL of a mixed solvent of acetonitrile and water (V : V = 6 : 1). Lithium hydroxide monohydrate (145 mg, 3.46 mmol) was added. The mixture was stirred at 40 °C. for 18 h. The reaction mixture was cooled to room temperature, adjusted to pH 5-6 with aqueous citric acid solution (1 M) and extracted with ethyl acetate (30 mL×3).The organic phase was concentrated under reduced pressure and then purified by high performance liquid chromatography (Gilson281, column: Boston Phlex C18 150×30 mm, 5 µm; mobile phase 1: water (containing 10 mmol/L ammonium bicarbonate); mobile phase 2: acetonitrile; 15 min of gradient elution: 30% to 50%, flow rate: 30 mL/min) to give the title product **A** (310 mg, yield: 76.46%).

MS m/z (ESI):592.2 [M+1].

**¹** H NMR (500 MHz, DMSO-d₆) δ 12.42-12.97 (brs, 1H), 8.20-8.28 (m, 1H), 7.74-7.83 (m, 1H), 7.61 (d, 1H), 7.55-7.58 (m, 1H), 7.48-7.54 (m, 1H), 7.38-7.44 (m, 1H), 6.70-6.90 (m, 3H), 5.40-7.49 (m, 1H), 5.01-5.13 (m, 1H), 4.72-4.84 (m, 1H), 4.59-4.67 (m, 1H), 4.39-4.51 (m, 2H), 4.31-4.38 (m, 1H), 4.04-4.13 (m, 1H), 3.86-3.95 (m, 1H), 3.71-3.79 (m, 1H), 2.91-3.01 (m, 1H), 2.77-2.88 (m, 2H), 2.61-2.72 (m, 1H), 2.33-2.44 (m, 1H), 2.07-2.25 (m, 2H), 1.73-1.81 (m, 1H), 1.63-1.73 (m, 2H), 1.54-1.63 (m, 1H).

### Test Example 1:Evaluation of Agonist Activity Against GLP-1 Receptor

### I. Purpose

This experiment was intended to test the agonist activity of the compound molecules against the GLP-1 receptor and evaluate the in vitro activity of the molecules according to EC₅₀. The experiment adopted a ONE-Glo^{™} Luciferase Assay System (Promega, E6110). Under the action of compound molecules, GLP-1R downstream signaling pathways were activated to cause elevated cAMP level. The combination of cAMP and CRE could start the transcription expression of CRE downstream luciferase genes, the luciferase could emit fluorescence when reacting with substrates thereof, and the activity of the compound for agonizing GLP-1 receptors was reflected by measuring fluorescence signals through a ONE-Glo^{™} reagent.

### II. Method

Stably-expressed CHO-K1/CRE-luc/GLP-1 receptor cell strains (self-construction of GLP-1 receptor plasmid; CRE-luc plasmid Promega E8471) were constructed. CHO-K1/CRE-luc/GLP-1 receptor cells were digested, and resuspended after centrifugation. Single cell suspension was uniformly mixed, and adjusted to a viable cell density of 2.5 × 10⁵ cells/mL with a cell culture medium (DME/F-12+10% FBS), and the resulting solution was added to a 96-well cell culture plate at 90 µL/well (Corning, #3903). The plate was incubated in an incubator for 16 h (37 °C., 5% CO₂).

The compound was dissolved in DMSO to prepare a stock solution with an initial concentration of 20 mM. The starting concentration of the small molecule compound was 0.2 mM, and the compound underwent 3-fold serial dilution for a total of 10 concentration points, with DMSO at the 11th point. To another 96-well plate was added 95 µL of cell culture medium (DME/F-12+10% FBS), 5 µL of test samples with different concentrations were added to each well, followed by uniform mixing, and then 10 µL of test samples with different concentrations were added to each well, with two duplicate wells set for each sample. The plate was incubated in an incubator for 6 h (37 °C., 5% CO₂). The 96-well cell culture plate was taken out, and 100 µL of ONE-Glo^{™} reagent was added to each well, followed by incubation at room temperature for 10 min. The plate was placed in a microplate reader (EnVision 2105, PE) for determination of chemiluminescence.

### III. Data Analysis

Data were processed and analyzed using Microsoft Excel and Graphpad Prism 5. EC₅₀ values of the compounds were obtained, and the results are shown in Table 1 below.

**Table 1**

| Compound | EC₅₀ (nM) | Emax% |
|---|---|---|
| A | 0.74 | 103.02 |

### Example 2: Amorphous form of tromethamine salt of Compound A

15 mg of Compound A was weighed, 0.3 mL of water was added, the mixture was stirred, 3.38 mg of tromethamine was added, and 3 cycles of heating and cooling from 50 °C to 5 °C at 0.8 K/min were carried out, and after centrifugation, the supernatant was slowly volatilized to obtain a solid. After detection by X-ray powder diffraction, the product was an amorphous form of a tromethamine salt, and the XRPD pattern was as shown in FIG. 1.

### Example 3: Preparation of crystal form A of tromethamine salt of Compound A

10 mg of Compound A was weighed, 200 µl of ethanol was added, the mixture was stirred, and 2.23 mg of tromethamine solid was added, and the mixture was stirred, centrifuged, and dried to obtain a solid. After detection by X-ray powder diffraction, the XRPD pattern was as shown in FIG. 2, and the positions of the characteristic peaks thereof were as shown in Table 2. The product was designated as crystal form A of the tromethamine salt. The DSC pattern showed that the peak values of endothermic peaks were 163.02 °C and 174.83 °C. The TGA pattern showed that the weight loss at 30-190 °C was 2.38%.

**Table 2**

| **Peak number** | **2θ value [° or degrees]** | **d[Å]** | **Relative intensity %** |
|---|---|---|---|
| 1 | 6.850 | 12.89457 | 100 |
| 2 | 8.051 | 10.97333 | 4.1 |
| 3 | 9.982 | 8.85433 | 9.7 |
| 4 | 12.697 | 6.96639 | 7.6 |
| 5 | 13.789 | 6.41715 | 27.4 |
| 6 | 14.704 | 6.01968 | 2.6 |
| 7 | 14.990 | 5.90531 | 2.9 |
| 8 | 15.763 | 5.61761 | 9.1 |
| 9 | 16.148 | 5.48451 | 12.8 |
| 10 | 18.016 | 4.91975 | 6.9 |
| 11 | 19.016 | 4.66326 | 6.9 |
| 12 | 20.109 | 4.41207 | 3.2 |
| 13 | 22.138 | 4.01217 | 13.9 |
| 14 | 23.868 | 3.72507 | 3.8 |
| 15 | 25.712 | 3.46204 | 4.6 |
| 16 | 27.909 | 3.19426 | 5.7 |
| 17 | 28.988 | 3.07781 | 3.5 |
| 18 | 31.535 | 2.83478 | 4.2 |
| 19 | 35.145 | 2.55140 | 2.7 |

### Example 4: Preparation of crystal form A of tromethamine salt of Compound A

10 mg of Compound A was weighed, 200 µl of ethyl acetate was added, the mixture was stirred, and 2.23 mg of tromethamine solid was added, and the mixture was stirred, centrifuged, and dried to obtain a solid. After detection by X-ray powder diffraction, the product was crystal form A of the tromethamine salt.

### Example 5: Preparation of crystal form A of tromethamine salt of Compound A

10 mg of Compound A was weighed, 200 µl of methyl tert-butyl ether (MTBE) was added, the mixture was stirred, and 2.23 mg of tromethamine solid was added, and the mixture was stirred, centrifuged, and dried to obtain a solid. After detection by X-ray powder diffraction, the product was crystal form A of the tromethamine salt.

### Example 6: Preparation of crystal form A of tromethamine salt of Compound A

10 mg of Compound A was weighed, 200 µl of a mixed solution of tetrahydrofuran/methyl tert-butyl ether (THF : MTBE = 1 : 2) was added, the mixture was stirred, and 2.23 mg of tromethamine solid was added, and the mixture was stirred, centrifuged, and dried to obtain a solid. After detection by X-ray powder diffraction, the product was crystal form A of the tromethamine salt.

### Example 7: Preparation of crystal form A of tromethamine salt of Compound A

150 mg of Compound A was weighed, 3 mL of anhydrous ethanol was added, the mixture was heated to 75 °C, a turbid solution of tromethamine (33.7 mg, dissolved in 5 mL of anhydrous ethanol) was dropwise added and dissolved until clear, a small amount of a seed crystal of crystal form A (prepared by referring to the method in Example 3) was added, the mixture was stirred and subjected to crystallization, and the resulting product was cooled to room temperature, stirred, filtered and dried. After detection by X-ray powder diffraction, the product was crystal form A of the tromethamine salt.

### Example 8: Preparation of crystal form B of tromethamine salt of Compound A

15 mg of Compound A was weighed, 0.4 mL of 10% water-acetone was added, the mixture was stirred, 3.38 mg of tromethamine was added, and the mixture was dissolved until clear, placed at 4 °C, stirred, precipitated, centrifuged, and dried to obtain a solid. After detection by X-ray powder diffraction, the XRPD pattern was as shown in FIG. 3, and the positions of the characteristic peaks thereof were as shown in Table 3. The product was designated as crystal form B of the tromethamine salt. The DSC pattern showed that the peak values of endothermic peaks were 128.76 °C and 160.93 °C. The TGA pattern showed that the weight loss at 30-140 °C was 3.39%.

**Table 3**

| **Peak number** | **2θ value [° or degrees]** | **d[Å]** | **Relative intensity %** |
|---|---|---|---|
| 1 | 8.144 | 10.84814 | 100 |
| 2 | 10.511 | 8.40946 | 9.2 |
| 3 | 12.290 | 7.19601 | 46.5 |
| 4 | 13.996 | 6.32238 | 7.9 |
| 5 | 14.665 | 6.03564 | 6.4 |
| 6 | 15.607 | 5.67317 | 4.9 |
| 7 | 16.520 | 5.36188 | 4.2 |
| 8 | 18.883 | 4.69583 | 3.9 |
| 9 | 20.632 | 4.30140 | 21.9 |
| 10 | 21.699 | 4.09225 | 20.7 |
| 11 | 23.741 | 3.74475 | 2.6 |
| 12 | 24.743 | 3.59538 | 5.5 |
| 13 | 26.055 | 3.41725 | 5.4 |
| 14 | 27.035 | 3.29553 | 1.4 |

### Example 9: Preparation of crystal form C of tromethamine salt of Compound A

50 mg of a sample of crystal form A of a tromethamine salt of the compound was weighed into 1 mL of methyl tert-butyl ether to form a suspension, the suspension was stirred at room temperature for 72 hours and filtered, and the filter cake was collected and dried in vacuum to obtain a solid. After detection by X-ray powder diffraction, the XRPD pattern was as shown in FIG. 4, and the positions of the characteristic peaks thereof were as shown in Table 4. The product was designated as crystal form C of the tromethamine salt. The DSC pattern showed that the peak value of an endothermic peak was 158.9 °C. The TGA pattern showed that the weight loss at 30-150 °C was 5.50%.

**Table 4**

| **Peak number** | **2θ value [° or degrees]** | **d[Å]** | **Relative intensity %** |
|---|---|---|---|
| 1 | 6.551 | 13.48213 | 100.0 |
| 2 | 8.392 | 10.52791 | 12.1 |
| 3 | 9.269 | 9.53396 | 53.4 |
| 4 | 13.175 | 6.71450 | 58.2 |
| 5 | 14.802 | 5.98007 | 27.8 |
| 6 | 15.864 | 5.58188 | 20.8 |
| 7 | 16.906 | 5.24026 | 42.6 |
| 8 | 18.776 | 4.72233 | 39.7 |
| 9 | 20.430 | 4.34365 | 38.0 |
| 10 | 22.211 | 3.99915 | 12.8 |
| 11 | 22.922 | 3.87669 | 16.0 |
| 12 | 23.574 | 3.77095 | 10.6 |
| 13 | 25.496 | 3.49088 | 19.9 |
| 14 | 26.290 | 3.38714 | 9.9 |
| 15 | 26.770 | 3.32756 | 5.9 |
| 16 | 28.208 | 3.16109 | 4.7 |
| 17 | 29.846 | 2.99124 | 7.1 |
| 18 | 31.404 | 2.84630 | 5.2 |

### Example 10: Preparation of crystal form C of tromethamine salt of Compound A

50 mg of a sample of crystal form A of a tromethamine salt of the compound was weighed and added to 1 mL of methyl tert-butyl ether to form a suspension, the suspension was stirred at room temperature for 72 h and filtered, and the filter cake was collected and dried in vacuum to obtain a solid. After detection by X-ray powder diffraction, the product was crystal form C of the tromethamine salt.

### Example 11: Preparation of crystal form D of tromethamine salt of Compound A

15 mg of Compound A was weighed, 0.3 mL of methanol was added, the mixture was stirred, 3.38 mg of tromethamine was added, and 3 cycles of heating and cooling from 50 °C to 5 °C at 0.8 K/min were carried out, and after centrifugation and drying, a solid was obtained. After detection by X-ray powder diffraction, the XRPD pattern was as shown in FIG. 5, and the positions of the characteristic peaks thereof were as shown in Table 5. The product was designated as crystal form D of the tromethamine salt.

**Table 5**

| **Peak number** | **2θ value [° or degrees]** | **d[Å]** | **Relative intensity %** |
|---|---|---|---|
| 1 | 5.281 | 16.72183 | 54.0 |
| 2 | 9.912 | 8.91689 | 35.8 |
| 3 | 10.292 | 8.58779 | 60.4 |
| 4 | 10.961 | 8.06570 | 29.8 |
| 5 | 11.613 | 7.61366 | 17.0 |
| 6 | 13.322 | 6.64067 | 100.0 |
| 7 | 15.351 | 5.76743 | 17.7 |
| 8 | 17.586 | 5.03916 | 7.6 |
| 9 | 18.283 | 4.84857 | 17.7 |
| 10 | 19.214 | 4.61564 | 18.0 |
| 11 | 19.890 | 4.46030 | 5.8 |
| 12 | 21.390 | 4.15078 | 58.6 |
| 13 | 22.215 | 3.99846 | 32.3 |
| 14 | 23.471 | 3.78721 | 8.4 |
| 15 | 23.979 | 3.70818 | 28.7 |
| 16 | 25.029 | 3.55493 | 21.1 |
| 17 | 25.846 | 3.44438 | 24.3 |
| 18 | 26.741 | 3.33110 | 7.4 |
| 19 | 27.232 | 3.27207 | 6.6 |
| 20 | 27.918 | 3.19320 | 11.2 |
| 21 | 29.536 | 3.02186 | 5.9 |
| 22 | 30.121 | 2.96451 | 13.0 |
| 23 | 30.550 | 2.92385 | 7.1 |
| 24 | 32.240 | 2.77436 | 0.7 |

### Example 12: Preparation of crystal form D of tromethamine salt of Compound A

1.5 g of Compound A was weighed into 18 mL of isopropanol, heated to 75 °C, an aqueous solution of tromethamine (308 mg, dissolved in 0.77 mL of water) was added dropwise, 6 mL of isopropanol and 12 mL of methanol were added, the mixture was stirred at 75 °C for half an hour to precipitate a solid, slowly cooled, stirred at room temperature for 16 h, stirred in an ice bath for half an hour, and after filtration, a filter cake core was collected and dried to obtain a solid. After detection by X-ray powder diffraction, the product was crystal form D of the tromethamine salt.

### Example 13: Preparation of crystal form E of tromethamine salt of Compound A

60 mg of Compound A was weighed, 1 ml of acetonitrile was added, and 15.7 mg of tromethamine solid was added, the mixture was stirred and crystallized, and after centrifugation and drying, a solid was obtained. After detection by X-ray powder diffraction, the XRPD pattern was as shown in FIG. 6, and the positions of the characteristic peaks thereof were as shown in Table 6. The product was designated as crystal form E of the tromethamine salt. The DSC pattern showed that the peak values of endothermic peaks were 127.83 °C and 160.76 °C. The TGA pattern showed that the weight loss at 30-190 °C was 2.13%.

**Table 6**

| **Peak number** | **2θ value [° or degrees]** | **d[Å]** | **Relative intensity %** |
|---|---|---|---|
| 1 | 6.821 | 12.94824 | 100.0 |
| 2 | 10.035 | 8.80744 | 12.2 |
| 3 | 12.653 | 6.99062 | 12.2 |
| 4 | 13.727 | 6.44565 | 24.0 |
| 5 | 14.787 | 5.98596 | 13.1 |
| 6 | 16.081 | 5.50705 | 11.2 |
| 7 | 16.648 | 5.32081 | 11.5 |
| 8 | 18.571 | 4.77389 | 9.9 |
| 9 | 19.451 | 4.56002 | 7.0 |
| 10 | 20.151 | 4.40308 | 11.1 |
| 11 | 20.736 | 4.28024 | 4.0 |
| 12 | 22.204 | 4.00035 | 10.8 |
| 13 | 25.386 | 3.50570 | 1.9 |
| 14 | 27.774 | 3.20945 | 5.8 |
| 15 | 29.941 | 2.98196 | 4.7 |

### Example 14: Preparation of crystal form F of tromethamine salt of Compound A

A sample of crystal form A of the tromethamine salt of Compound A was placed at 80 °C/0% RH for 96 h or dried at 167 °C. After detection by X-ray powder diffraction, the XRPD pattern was as shown in FIG. 7, and the positions of the characteristic peaks thereof were as shown in Table 7. The product was designated as crystal form F of the tromethamine salt. The DSC pattern showed that the peak values of endothermic peaks were 159.88 °C and 175.93 °C. The TGA pattern showed that the weight loss at 30-180 °C was 2.91%.

**Table 7**

| **Peak number** | **2θ value [° or degrees]** | **d[Å]** | **Relative intensity %** |
|---|---|---|---|
| 1 | 6.777 | 13.03260 | 15.1 |
| 2 | 7.674 | 11.51123 | 100.0 |
| 3 | 10.614 | 8.32815 | 27.1 |
| 4 | 11.594 | 7.62626 | 13.1 |
| 5 | 13.671 | 6.47190 | 5.5 |
| 6 | 14.408 | 6.14261 | 9.7 |
| 7 | 14.882 | 5.94792 | 18.4 |
| 8 | 16.400 | 5.40078 | 38.0 |
| 9 | 18.645 | 4.75531 | 69.7 |
| 10 | 20.849 | 4.25720 | 8.1 |
| 11 | 21.384 | 4.15185 | 7.6 |
| 12 | 21.731 | 4.08646 | 8.9 |
| 13 | 22.108 | 4.01748 | 8.0 |
| 14 | 23.021 | 3.86018 | 1.7 |
| 15 | 24.721 | 3.59845 | 7.3 |
| 16 | 26.169 | 3.40251 | 7.7 |
| 17 | 29.192 | 3.05676 | 7.9 |

### Example 15: Preparation of amorphous form of ammonium salt of Compound A

15 mg of Compound A was weighed, 0.3 mL of acetone was added, the mixture was stirred, 28 µL of 2 M ammonia aqueous solution was added, and 3 cycles of heating and cooling from 50 °C to 5 °C at 0.8 K/min were carried out, and after centrifugation, the supernatant was slowly volatilized to obtain a solid. After detection by X-ray powder diffraction, the X-ray powder diffraction pattern had no obvious characteristic peaks, and the product was an amorphous form of the amine salt.

### Example 16: Preparation of amorphous form of potassium salt of Compound A

10 mg of Compound A was weighed, 200 µL of a mixed solution of tetrahydrofuran/tert-butyl dimethyl ether (THF : MTBE = 1 : 2) was added, followed by 9.3 µL of 2 M potassium hydroxide aqueous solution, and the mixture was pulped at room temperature, centrifuged and dried to obtain a solid. After detection by X-ray powder diffraction, the X-ray powder diffraction pattern had no obvious characteristic peaks, and the product was an amorphous form of the potassium salt.

### Example 17: Preparation of crystal form α of potassium salt of Compound A

10 mg of Compound A was weighed, 200 µL of ethyl acetate was added, followed by 9.3 µL of 2 M potassium hydroxide aqueous solution, and the mixture was pulped at room temperature, centrifuged and dried to obtain a solid. After detection by X-ray powder diffraction, the product was crystal form α of the potassium salt, the XRPD pattern was as shown in FIG. 8, and the positions of the characteristic peaks thereof were as shown in Table 8. The DSC pattern showed that the peak values of endothermic peaks were 87.46 °C, 159.28 °C, and 230.06 °C. The TGA pattern showed that the weight loss at 30-150 °C was 5.17%.

**Table 8**

| **Peak number** | **2θ value [° or degrees]** | **d[Å]** | **Relative intensity %** |
|---|---|---|---|
| 1 | 9.564 | 9.23983 | 100.0 |
| 2 | 11.515 | 7.67827 | 65.3 |
| 3 | 14.683 | 6.02808 | 36.4 |
| 4 | 16.058 | 5.51498 | 26.4 |
| 5 | 17.033 | 5.20130 | 14.1 |
| 6 | 17.945 | 4.93920 | 4.7 |
| 7 | 18.859 | 4.70181 | 17.9 |
| 8 | 19.607 | 4.52406 | 59.2 |
| 9 | 20.391 | 4.35171 | 40.2 |
| 10 | 21.064 | 4.21428 | 11.3 |
| 11 | 22.592 | 3.93261 | 28.9 |
| 12 | 23.320 | 3.81134 | 16.1 |
| 13 | 24.449 | 3.63787 | 12.9 |
| 14 | 25.176 | 3.53444 | 19.8 |
| 15 | 25.933 | 3.43297 | 13.6 |
| 16 | 27.080 | 3.29008 | 12.7 |
| 17 | 27.708 | 3.21692 | 13.3 |

### Example 18: Preparation of crystal form α of potassium salt of Compound A

10 mg of Compound A was weighed, 200 µL of methyl tert-butyl ether was added, followed by 9.3 µL of 2 M potassium hydroxide aqueous solution, and the mixture was pulped at room temperature, centrifuged and dried to obtain a solid. After detection by X-ray powder diffraction, the product was crystal form α of the potassium salt.

### Example 19: Preparation of amorphous form of arginine salt of Compound A

15 mg of Compound A was weighed, 0.3 mL of methanol was added, the mixture was stirred, 9.72 mg of arginine was added, 3 cycles of heating and cooling from 50 °C to 5 °C at 0.8 K/min were carried out, and after centrifugation, the supernatant was slowly volatilized to obtain a solid. After detection by X-ray powder diffraction, the X-ray powder diffraction pattern had no obvious characteristic peaks, and the product was an amorphous form of the arginine salt.

### Example 20: Preparation of amorphous form of sodium salt of Compound A

10 mg of Compound A was weighed, 200 µL of methyl tert-butyl ether was added, followed by 9.3 µL of 2 M sodium hydroxide aqueous solution, and the mixture was pulped at room temperature, centrifuged and dried to obtain a solid. After detection by X-ray powder diffraction, the X-ray powder diffraction pattern had no obvious characteristic peaks, and the product was an amorphous form of the sodium salt.

### Example 21: Preparation of crystal form I of sodium salt of Compound A

15 mg of Compound A was weighed, 0.3 ml of methanol was added, 28 µL of 2 M sodium hydroxide aqueous solution was added, the mixture was stirred and dissolved, 3 cycles of heating and cooling from 50 °C to 5 °C at 0.8 K/min were carried out, and after drying, a solid was obtained. After detection by X-ray powder diffraction, the XRPD pattern was as shown in FIG. 9, and the positions of the characteristic peaks thereof were as shown in Table 9. The product was designated as crystal form I of the sodium salt. The DSC pattern showed that the peak values of endothermic peaks were 77.63 °C, 157.96 °C, 181.45 °C, and 232.77 °C. The TGA pattern showed that the weight loss at 30-150 °C was 3.05%.

**Table 9**

| **Peak number** | **2θ value [° or degrees]** | **d[Å]** | **Relative intensity %** |
|---|---|---|---|
| 1 | 5.257 | 16.79552 | 100.0 |
| 2 | 5.806 | 15.20931 | 94.7 |
| 3 | 6.795 | 12.99734 | 57.6 |
| 4 | 10.106 | 8.74583 | 63.2 |
| 5 | 12.203 | 7.24689 | 54.3 |
| 6 | 20.693 | 4.28891 | 59.0 |

### Example 22: Preparation of crystal form I of sodium salt of Compound A

10 mg of Compound A was weighed, 200 µL of ethyl acetate was added, followed by 9.3 µL of 2M sodium hydroxide aqueous solution; and if it was not dissolved clear, the mixture was stirred overnight at room temperature, centrifuged and dried to obtain a solid. After detection by X-ray powder diffraction, the product was crystal form I of the sodium salt.

### Example 23: Preparation of crystal form II of sodium salt of Compound A

15 mg of Compound A was weighed, 0.3 mL of acetone was added, 28 µL of 2 M sodium hydroxide aqueous solution was added, the mixture was stirred and dissolved, 3 cycles of heating and cooling from 50 °C to 5 °C at 0.8 K/min were carried out, and after centrifugation and drying, a solid was obtained. After detection by X-ray powder diffraction, the XRPD pattern was as shown in FIG. 10, and the positions of the characteristic peaks thereof were as shown in Table 10. The product was designated as crystal form II of the sodium salt. The DSC pattern showed that the peak values of endothermic peaks were 118.67 °C and 235.34 °C. The TGA pattern showed that the weight loss at 30-150 °C was 2.85%.

**Table 10**

| **Peak number** | **2θ value [° or degrees]** | **d[Å]** | **Relative intensity %** |
|---|---|---|---|
| 1 | 5.574 | 15.84162 | 65.2 |
| 2 | 9.754 | 9.06033 | 100.0 |
| 3 | 11.731 | 7.53794 | 85.6 |
| 4 | 14.856 | 5.95832 | 12.4 |
| 5 | 16.091 | 5.50357 | 12.0 |
| 6 | 19.730 | 4.49609 | 82.6 |
| 7 | 22.670 | 3.91923 | 5.7 |

### Example 24: Preparation of crystal form II of sodium salt of Compound A

15 mg of Compound A was weighed, and 0.3 mL of acetonitrile was added; and if it was not dissolved after stirring, 14 µL of 2 M sodium hydroxide aqueous solution was added for dissolution, 3 cycles of heating and cooling from 50 °C to 5 °C at 0.8 K/min were carried out, and after centrifugation and drying, a solid was obtained. After detection by X-ray powder diffraction, the product was crystal form II of the sodium salt.

### Example 25: Preparation of amorphous form of meglumine salt of Compound A

15 mg of Compound A was weighed, 0.3 mL of methanol was added, the mixture was stirred, 11.0 mg of meglumine was added, 3 cycles of heating and cooling from 50 °C to 5 °C at 0.8 K/min were carried out, and after centrifugation, the supernatant was slowly volatilized to obtain a solid. After detection by X-ray powder diffraction, the X-ray powder diffraction pattern had no obvious characteristic peaks, and the product was an amorphous form of the meglumine salt.

### Example 26: Preparation of crystal form I of meglumine salt of Compound A

15 mg of Compound A was weighed, 0.3 mL of acetonitrile was added, the mixture was stirred, 5.5 mg of meglumine was added, 3 cycles of heating and cooling from 50 °C to 5 °C at 0.8 K/min were carried out, and after centrifugation, the supernatant was slowly volatilized to obtain a solid. After detection by X-ray powder diffraction, the XRPD pattern was as shown in FIG. 11, and the positions of the characteristic peaks thereof were as shown in Table 11. The product was designated as crystal form I of the meglumine salt. The DSC pattern showed that the peak values of endothermic peaks were 60.00 °C and 104.48 °C. The TGA pattern showed that the weight loss at 30-110 °C was 0.18%.

**Table 11**

| **Peak number** | **2θ value [° or degrees]** | **d[Å]** | **Relative intensity %** |
|---|---|---|---|
| 1 | 5.498 | 16.06202 | 100.0 |
| 2 | 8.314 | 10.62603 | 6.1 |
| 3 | 8.901 | 9.92698 | 11.4 |
| 4 | 11.013 | 8.02776 | 23.2 |
| 5 | 11.891 | 7.43643 | 6.3 |
| 6 | 12.810 | 6.90505 | 7.4 |
| 7 | 14.626 | 6.05172 | 22.3 |
| 8 | 15.683 | 5.64601 | 6.7 |
| 9 | 15.931 | 5.55878 | 9.0 |
| 10 | 17.942 | 4.93981 | 20.1 |
| 11 | 18.748 | 4.72941 | 4.5 |
| 12 | 19.454 | 4.55917 | 12.9 |
| 13 | 20.393 | 4.35126 | 9.6 |
| 14 | 21.419 | 4.14519 | 8.3 |
| 15 | 22.234 | 3.99506 | 7.5 |
| 16 | 22.668 | 3.91953 | 13.7 |
| 17 | 23.391 | 3.79994 | 8.0 |
| 18 | 23.936 | 3.71469 | 2.8 |
| 19 | 24.739 | 3.59590 | 7.5 |
| 20 | 25.696 | 3.46406 | 11.9 |
| 21 | 26.682 | 3.33824 | 4.7 |
| 22 | 33.209 | 2.69554 | 7.0 |

### Example 27: Preparation of amorphous form of ethanolamine salt of Compound A

15 mg of Compound A was weighed, 0.3 mL of methanol was added, the mixture was stirred, 28 µL of 2 M ethanolamine aqueous solution was added, and 3 cycles of heating and cooling from 50 °C to 5 °C at 0.8 K/min were carried out, and after centrifugation, the supernatant was slowly volatilized to obtain a solid. After detection by X-ray powder diffraction, the X-ray powder diffraction pattern had no obvious characteristic peaks, and the product was an amorphous form of the ethanolamine salt.

### Example 28: Preparation of crystal form I of p-toluenesulfonate salt of Compound A

15 mg of Compound A was weighed, and 0.3 mL of acetonitrile was added; and if it was not dissolved after stirring, 9.61 mg of p-toluenesulfonic acid was added for dissolution, 3 cycles of heating and cooling from 50 °C to 5 °C at 0.8 K/min were carried out, and after centrifugation and drying, a solid was obtained. After detection by X-ray powder diffraction, the XRPD pattern was as shown in FIG. 12, and the positions of the characteristic peaks thereof were as shown in Table 12. The product was designated as crystal form I of the p-toluenesulfonate salt. The DSC pattern showed that the peak value of an endothermic peak was 191.14 °C. The TGA pattern showed that the weight loss at 30-150 °C was 0.93%.

**Table 12**

| **Peak number** | **2θ value [° or degrees]** | **d[Å]** | **Relative intensity %** |
|---|---|---|---|
| 1 | 5.453 | 16.19243 | 97.8 |
| 2 | 5.884 | 15.00779 | 66.0 |
| 3 | 8.063 | 10.95652 | 67.8 |
| 4 | 12.925 | 6.84366 | 61.8 |
| 5 | 13.825 | 6.40024 | 41.8 |
| 6 | 15.399 | 5.74960 | 45.5 |
| 7 | 16.071 | 5.51041 | 59.5 |
| 8 | 16.560 | 5.34900 | 39.4 |
| 9 | 17.066 | 5.19150 | 25.2 |
| 10 | 18.231 | 4.86231 | 47.5 |
| 11 | 19.778 | 4.48533 | 100.0 |
| 12 | 20.861 | 4.25472 | 33.0 |
| 13 | 21.917 | 4.05212 | 54.6 |
| 14 | 23.898 | 3.72055 | 31.2 |
| 15 | 24.467 | 3.63525 | 12.8 |
| 16 | 26.744 | 3.33064 | 36.5 |
| 17 | 28.136 | 3.16898 | 22.4 |

### Example 29: Preparation of crystal form I of tartrate salt of Compound A

10 mg of Compound A was weighed, and 200 µL of ethanol was added; and if it was not dissolved clear, 9.3 µL of 2 M L-tartaric acid aqueous solution was further added, and the mixture was stirred, centrifuged and dried to obtain a solid. After detection by X-ray powder diffraction, the XRPD pattern was as shown in FIG. 13, and the positions of the characteristic peaks thereof were as shown in Table 13. The product was designated as crystal form I of the tartrate salt. The DSC pattern showed that the peak values of endothermic peaks were 115.94 °C and 138.28 °C. The TGA pattern showed that the weight loss at 30-110 °C was 2.60%.

**Table 13**

| **Peak number** | **2θ value [° or degrees]** | **d[Å]** | **Relative intensity %** |
|---|---|---|---|
| 1 | 9.342 | 9.45908 | 12.3 |
| 2 | 10.132 | 8.72312 | 12.8 |
| 3 | 10.858 | 8.14155 | 13.7 |
| 4 | 11.272 | 7.84361 | 15.1 |
| 5 | 12.810 | 6.90483 | 44.8 |
| 6 | 14.448 | 6.12567 | 29.5 |
| 7 | 15.604 | 5.67430 | 28.0 |
| 8 | 18.824 | 4.71037 | 100.0 |
| 9 | 20.410 | 4.34785 | 29.1 |
| 10 | 21.890 | 4.05711 | 36.9 |
| 11 | 24.472 | 3.63456 | 39.4 |
| 12 | 27.880 | 3.19747 | 15.9 |

### Example 30: Preparation of crystal form I of tartrate salt of Compound A

10 mg of Compound A was weighed, 200 µL of ethyl acetate was added, followed by 9.3 µL of 2 M L-tartaric acid aqueous solution, and the mixture was stirred, centrifuged and dried to obtain a solid. After detection by X-ray powder diffraction, the product was crystal form I of the tartrate salt.

### Example 31: Preparation of crystal form I of tartrate salt of Compound A

10 mg of Compound A was weighed, 200 µL of methyl tert-butyl ether was added, followed by 9.3 µL of 2 M L-tartaric acid aqueous solution, and the mixture was stirred, centrifuged and dried to obtain a solid. After detection by X-ray powder diffraction, the product was crystal form I of the tartrate salt.

### Example 32: Preparation of amorphous form of sulfate salt of Compound A

15 mg of Compound A was weighed, 0.3 mL of acetonitrile was added, the mixture was stirred, 28 µL of 2 M sulfuric acid was added, 3 cycles of heating and cooling from 50 °C to 5 °C at 0.8 K/min were carried out, and after centrifugation, the supernatant was slowly volatilized to obtain a solid. After detection by X-ray powder diffraction, the X-ray powder diffraction pattern had no obvious characteristic peaks, and the product was an amorphous form of the sulfate salt.

### Example 33: Preparation of crystal form α of malate salt of Compound A

10 mg of Compound A was weighed, and 200 µL of ethyl acetate was added; and if it was not dissolved clear, 9.3 µL of 2 M L-malic acid aqueous solution was further added, and the mixture was stirred, centrifuged and dried to obtain a solid. After detection by X-ray powder diffraction, the XRPD pattern was as shown in FIG. 14, and the positions of the characteristic peaks thereof were as shown in Table 15. The product was designated as crystal form α of the malate salt. The DSC pattern showed that the peak values of endothermic peaks were 56.15 °C, 98.97 °C, and 126.79 °C. The TGA pattern showed that the weight loss at 30-130 °C was 1.75%.

**Table 14**

| **Peak number** | **2θ value [° or degrees]** | **d[Å]** | **Relative intensity %** |
|---|---|---|---|
| 1 | 9.364 | 9.43652 | 10.9 |
| 2 | 10.050 | 8.79438 | 48.7 |
| 3 | 10.818 | 8.17138 | 36.2 |
| 4 | 11.213 | 7.88494 | 38.3 |
| 5 | 12.871 | 6.87232 | 27.2 |
| 6 | 13.774 | 6.42394 | 17.4 |
| 7 | 14.508 | 6.10037 | 46.1 |
| 8 | 15.693 | 5.64245 | 46.3 |
| 9 | 17.760 | 4.99004 | 11.9 |
| 10 | 18.924 | 4.68576 | 100.0 |
| 11 | 20.240 | 4.38383 | 53.9 |
| 12 | 21.829 | 4.06820 | 39.9 |
| 13 | 24.396 | 3.64574 | 18.5 |
| 14 | 25.100 | 3.54504 | 4.8 |
| 15 | 26.200 | 3.39860 | 12.3 |
| 16 | 27.936 | 3.19122 | 12.1 |
| 17 | 28.188 | 3.16326 | 10.4 |
| 18 | 30.508 | 2.92776 | 4.8 |
| 19 | 30.983 | 2.88400 | 5.6 |

### Example 34: Preparation of crystal form α of malate salt of Compound A

10 mg of Compound A was weighed, 200 µL of MTBE was added, followed by 9.3 µL of 2 M L-malic acid aqueous solution, and the mixture was stirred, centrifuged and dried to obtain a solid. After detection by X-ray powder diffraction, the product was crystal form α of the malate salt.

### Example 35: Preparation of crystal form I of hydrochloride salt of Compound A

100 mg of Compound A was weighed, 1545 µl of 0.12 M hydrochloric acid aqueous solution was added, and the suspension was stirred overnight at room temperature, centrifuged and dried to obtain a solid. After detection by X-ray powder diffraction, the XRPD pattern was as shown in FIG. 15, and the positions of the characteristic peaks thereof were as shown in Table 15. The product was designated as crystal form I of the hydrochloride salt. The DSC pattern showed that the peak value of an endothermic peak was 163.16 °C. The TGA pattern showed that the weight loss at 30-170 °C was 2.50%.

**Table 15**

| **Peak number** | **2θ value [° or degrees]** | **d[Å]** | **Relative intensity %** |
|---|---|---|---|
| 1 | 9.939 | 8.89213 | 72.0 |
| 2 | 12.267 | 7.20959 | 16.9 |
| 3 | 13.123 | 6.74112 | 32.2 |
| 4 | 13.649 | 6.48262 | 33.9 |
| 5 | 14.333 | 6.17479 | 74.2 |
| 6 | 14.933 | 5.92798 | 93.6 |
| 7 | 16.616 | 5.33109 | 30.7 |
| 8 | 17.523 | 5.05706 | 90.9 |
| 9 | 18.480 | 4.79725 | 100.0 |
| 10 | 19.378 | 4.57689 | 39.7 |
| 11 | 20.134 | 4.40665 | 60.8 |
| 12 | 20.988 | 4.22925 | 34.1 |
| 13 | 22.377 | 3.96978 | 24.6 |
| 14 | 23.002 | 3.86339 | 30.5 |
| 15 | 24.477 | 3.63378 | 29.8 |
| 16 | 25.322 | 3.51441 | 30.5 |
| 17 | 26.399 | 3.37349 | 58.0 |
| 18 | 26.970 | 3.30335 | 47.7 |
| 19 | 27.609 | 3.22828 | 17.3 |
| 20 | 30.822 | 2.89865 | 23.3 |
| 21 | 32.119 | 2.78450 | 6.4 |
| 22 | 33.760 | 2.65286 | 17.7 |
| 23 | 25.541 | 2.52388 | 7.1 |

### Test Example 2: Study on hygroscopicity of crystal forms of salts of Compound A

Surface Measurement Systems intrinsic DVS was used at 25 °C. The observed humidity ranged from 0% to 95%. The step was 10%. The criterion was that the mass change dM/dT for each step was less than 0.002%. TMAX was 360 min. Two cycles were carried out.

**Table 16**

| Sample to be tested | 0.0% RH to 80.0% RH | Crystal form |
|---|---|---|
| Crystal form A of tromethamine salt | 5.02% | Unchanged |
| Crystal form D of tromethamine salt | 2.45% | Unchanged |
| Crystal form E of tromethamine salt | 7.27% | Unchanged |
| Crystal form I of tartrate salt | 1.44% | Unchanged |
| Crystal form α of malate salt | 2.45% | Unchanged |

### Test Example 3: Study on stability of crystal forms of salts of Compound A

A salt-type sample of Compound A was placed open and spread, and the stability of the sample was investigated under the conditions of illumination (4500 Lux), high temperature (40 °C and 60 °C) and high humidity (75% RH and 92.5% RH), respectively. The sampling and investigation period was 30 days.

**Table 17. Stability data of crystal form A of tromethamine salt**

| Condition | Time (days) | %Purity | Crystal form |
|---|---|---|---|
| Initial condition | 0 | 99.5 | Crystal form A of tromethamine salt |
| Illumination (4500 Lux) | 7 | 98.9 | Unchanged |
| | 14 | 97.6 | Unchanged |
| | 30 | 90.8 | Unchanged |
| 40 °C | 7 | 96.1 | Unchanged |
| | 14 | 91.7 | Unchanged |
| | 30 | 87.4 | Unchanged |
| 60 °C | 7 | 93.5 | Unchanged |
| | 14 | 88.6 | Unchanged |
| 75% RH | 7 | 99.4 | Unchanged |
| | 14 | 99.4 | Unchanged |
| | 30 | 99.3 | Unchanged |
| 92.5% RH | 7 | 99.3 | Unchanged |
| | 14 | 99.0 | Unchanged |
| | 30 | 98.6 | Unchanged |

**Table 18. Stability data of crystal form D of tromethamine salt**

| Condition | Time (days) | %Purity | Crystal form |
|---|---|---|---|
| Initial condition | 0 | 99.7 | Crystal form D of tromethamine salt |
| Illumination (4500 Lux) | 7 | 99.6 | Unchanged |
| | 14 | 99.6 | Unchanged |
| | 30 | 99.6 | Unchanged |
| 40 °C | 7 | 99.5 | Unchanged |
| | 14 | 99.3 | Unchanged |
| | 30 | 99.3 | Unchanged |
| 60 °C | 7 | 99.4 | Unchanged |
| | 14 | 99.1 | Unchanged |
| | 30 | 99.0 | Unchanged |
| 75% RH | 7 | 99.7 | Unchanged |
| | 14 | 99.6 | Unchanged |
| | 30 | 99.6 | Unchanged |
| 92.5% RH | 7 | 99.5 | Unchanged |
| | 14 | 99.4 | Unchanged |
| | 30 | 99.0 | Unchanged |

**Table 19. Stability data of crystal form E of tromethamine salt**

| Condition | Time (days) | %Purity | Crystal form |
|---|---|---|---|
| Initial condition | 0 | 98.3 | Crystal form E of tromethamine salt |
| Illumination (4500 Lux) | 7 | 98.1 | Unchanged |
| | 14 | 97.9 | Unchanged |
| | 30 | 95.5 | Unchanged |
| 40 °C | 7 | 96.0 | Unchanged |
| | 14 | 93.6 | Unchanged |
| | 30 | 85.0 | Unchanged |
| 60 °C | 7 | 94.8 | Unchanged |
| | 30 | 81.8 | Amorphous form |
| 75% RH | 7 | 98.3 | Unchanged |
| | 14 | 98.4 | Unchanged |
| | 30 | 98.3 | Unchanged |
| 92.5% RH | 7 | 98.2 | Unchanged |
| | 14 | 98.2 | Unchanged |
| | 30 | 98.1 | Amorphous form |

**Table 20. Stability data of crystal form I of hydrochloride salt**

| Condition | Time (days) | %Purity | Crystal form |
|---|---|---|---|
| Initial condition | 0 | 98.0 | Crystal form I of hydrochloride salt |
| Illumination (4500 Lux) | 5 | 97.9 | Unchanged |
| | 10 | 98.1 | Unchanged |
| | 30 | 98.0 | Unchanged |
| 40 °C | 5 | 98.0 | Unchanged |
| | 10 | 98.1 | Unchanged |
| | 30 | 97.8 | Unchanged |
| 60 °C | 5 | 97.6 | Unchanged |
| | 10 | 97.7 | Unchanged |
| | 30 | 97.7 | Unchanged |
| 75% RH | 5 | 98.1 | Unchanged |
| | 10 | 98.0 | Unchanged |
| | 30 | 98.1 | Unchanged |
| 92.5% RH | 5 | 98.1 | Unchanged |
| | 10 | 97.4 | Unchanged |
| | 30 | 96.9 | Unchanged |

**Table 21. Stability data of crystal form α of malate salt**

| Condition | Time (days) | %Purity | Crystal form |
|---|---|---|---|
| Initial condition | 0 | 99.0 | Crystal form α of malate salt |
| Illumination (4500 Lux) | 5 | 98.9 | Unchanged |
| | 10 | 98.8 | Unchanged |
| | 30 | 98.7 | Unchanged |
| 40 °C | 5 | 98.7 | Unchanged |
| | 10 | 98.6 | Unchanged |
| | 30 | 97.2 | Unchanged |
| 60 °C | 5 | 97.9 | Unchanged |
| | 10 | 97.7 | Unchanged |
| | 30 | 97.2 | Unchanged |
| 75% RH | 5 | 98.9 | Unchanged |
| | 10 | 98.9 | Unchanged |
| | 30 | 98.7 | Unchanged |
| 92.5% RH | 5 | 99.0 | Unchanged |
| | 10 | 98.9 | Unchanged |
| | 30 | 98.8 | Unchanged |

**Table 22. Stability data of crystal form I of tartrate salt**

| Condition | Time (days) | %Purity | Crystal form |
|---|---|---|---|
| Initial condition | 0 | 98.8 | Crystal form I of tartrate salt |
| Illumination (4500 Lux) | 5 | 98.7 | Unchanged |
| | 10 | 98.6 | Unchanged |
| | 30 | 98.3 | Unchanged |
| 40 °C | 5 | 98.4 | Unchanged |
| | 10 | 98.1 | Unchanged |
| | 30 | 97.6 | Unchanged |
| 60 °C | 5 | 97.9 | Unchanged |
| | 10 | 97.2 | Unchanged |
| | 30 | 96.4 | Unchanged |
| 75% RH | 5 | 98.6 | Unchanged |
| | 10 | 98.6 | Unchanged |
| | 30 | 98.4 | Unchanged |
| 92.5% RH | 5 | 98.7 | Unchanged |
| | 10 | 98.6 | Unchanged |
| | 30 | 98.3 | Unchanged |

Conclusion: The crystal form D of the tromethamine salt, the crystal form I of the hydrochloride salt, the crystal form α of the malate salt, and the crystal form I of the tartrate salt had good physical and chemical stability after standing for 30 days under the conditions of influencing factors; in addition, the crystal form A of the tromethamine salt had stable physical properties, the crystal form E of the tromethamine salt had slightly poor physical stability under high temperature and high humidity conditions, and the crystal forms A and E of the tromethamine salt had slightly poor chemical stability under high temperature conditions, but they could all meet the basic stability requirements of later formulation.

### Test Example 4: Long-term/accelerated stability of crystal forms of salts of Compound A

A salt-type sample was sealed with an aluminum foil bag and placed under the conditions of 25 °C/60% RH and 40 °C/75% RH, respectively, to investigate the stability.

**Table 23**

| | | | | | | |
|---|---|---|---|---|---|---|
| | Placement condition | %Purity | | | | Crystal form |
| Crystal form A of tromethamine salt | | Initial condition | 1 month | 2 months | 3 months | / |
| | 25 °C/60% RH | 99.8 | 99.7 | 99.8 | 99.8 | Unchanged |
| | 40 °C/75% RH | | 99.6 | 99.6 | 99.6 | Unchanged |
| Crystal form D of tromethamine salt | Placement condition | %Purity | | | | Crystal form |
| | | Initial condition | 1 month | 2 months | 3 months | / |
| | 25 °C/60% RH | 99.7 | 99.7 | / | 99.7 | Unchanged |
| | 40 °C/75% RH | | 99.5 | / | 99.1 | Unchanged |
| Crystal form E of tromethamine salt | Placement condition | %Purity | | | | Crystal form |
| | | Initial condition | 1 month | 2 months | 3 months | / |
| | 25 °C/60% RH | 98.3 | 98.2 | 98.3 | 98.1 | Unchanged |
| | 40 °C/75% RH | | 97.7 | 97.7 | 97.2 | Unchanged |
| Crystal form I of hydrochloride salt | Placement condition | %Purity | | | | Crystal form |
| | | Initial condition | 1 month | 2 months | 3 months | / |
| | 25 °C/60% RH | 98.0 | 98.1 | 97.9 | / | Unchanged |
| | 40 °C/75% RH | | 97.8 | 97.8 | / | Unchanged |
| Crystal form α of malate salt | Placement condition | %Purity | | | | Crystal form |
| | | Initial condition | 1 month | 2 months | 3 months | / |
| | 25 °C/60% RH | 99.0 | 98.8 | / | 98.7 | Unchanged |
| | 40 °C/75% RH | | 98.3 | / | 97.1 | Unchanged |
| Crystal form I of tartrate salt | Placement condition | %Purity | | | | Crystal form |
| | | Initial condition | 1 month | 2 months | 3 months | / |
| | 25 °C/60% RH | 98.8 | 98.4 | / | 98.2 | Unchanged |
| | 40 °C/75% RH | | 97.4 | / | 96.4 | Unchanged |

Conclusion: The long-term/accelerated stability experiment showed that the crystal forms A and D of the tromethamine salt and the crystal form I of the hydrochloride salt had good physical and chemical stability under long-term and accelerated conditions, and the crystal form E of the tromethamine salt and the crystal form I of the tartrate salt had good physical and chemical stability under long-term conditions.

## Claims

1. A pharmaceutically acceptable salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein the pharmaceutically acceptable salt is selected from a tromethamine salt, an ammonium salt, a potassium salt, an arginine salt, a sodium salt, a meglumine salt, an ethanolamine salt, a p-toluenesulfonate salt, a tartrate salt, a sulfate salt, a malate salt, and a hydrochloride salt.

2. The pharmaceutically acceptable salt according to claim 1, wherein the chemical ratio of the compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid to an alkali molecule or an acid molecule is 1 : 0.5 to 1 : 3, preferably 1 : 0.5, 1 : 1, 1 : 2, or 1 : 3, most preferably 1 : 1 or 1 : 2.

3. Crystal form A of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein an X-ray powder diffraction pattern of the crystal form A, obtained using Cu-Kα radiation (λ=1.5406 Å) and expressed with diffraction angle 2*θ*, has characteristic peaks at 6.850, 9.982, 13.789, 16.148, and 22.138; preferably characteristic peaks at 6.850, 9.982, 12.697, 13.789, 15.763, 16.148, 18.016, 19.016, and 22.138; more preferably characteristic peaks at 6.850, 8.051, 9.982, 12.697, 13.789, 14.704, 14.990, 15.763, 16.148, 18.016, 19.016, 20.109, 22.138, 25.712, and 27.909,
wherein the error range of the 2*θ* values is ± 0.2.

4. Crystal form B of a tromethamine salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein an X-ray powder diffraction pattern of the crystal form B, obtained using Cu-Kα radiation (λ=1.5406 Å) and expressed with diffraction angle 2*θ*, has characteristic peaks at 8.144, 10.511, 12.290, 20.632, and 21.699; preferably characteristic peaks at 8.144, 10.511, 12.290, 13.996, 14.665, 20.632, 21.699, and 24.743; more preferably characteristic peaks at 8.144, 10.511, 12.290, 13.996, 14.665, 15.607, 16.520, 18.883, 20.632, 21.699, 23.741, 24.743, 26.055, and 27.035,
wherein the error range of the 2*θ* values is ± 0.2.

5. Crystal form C of a tromethamine salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein an X-ray powder diffraction pattern of the crystal form C, obtained using Cu-Kα radiation (λ=1.5406 Å) and expressed with diffraction angle 2*θ*, has characteristic peaks at 6.551, 9.269, 13.175, and 16.906; preferably characteristic peaks at 6.551, 9.269, 13.175, 14.802, 15.864, 16.906, 18.776, 20.430, and 25.496; more preferably characteristic peaks at 6.551, 8.392, 9.269, 13.175, 14.802, 15.864, 16.906, 18.776, 20.430, 22.211, 22.922, 23.574, 25.496, and 26.290,
wherein the error range of the 2*θ* values is ± 0.2.

6. Crystal form D of a tromethamine salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein (4) an X-ray powder diffraction pattern of the crystal form D, obtained using Cu-Kα radiation (λ=1.5406 Å) and expressed with diffraction angle 2*θ*, has characteristic peaks at 5.281, 10.292, 13.322, and 21.390; preferably characteristic peaks at 5.281, 9.912, 10.292, 10.961, 13.322, 21.390, 22.215, 23.979, 25.029, and 25.846; more preferably characteristic peaks at 5.281, 9.912, 10.292, 10.961, 11.613, 13.322, 15.351, 18.283, 19.214, 21.390, 22.215, 23.471, 23.979, 25.029, 25.846, 27.918, and 30.121,
wherein the error range of the 2*θ* values is ± 0.2.

7. Crystal form E of a tromethamine salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein an X-ray powder diffraction pattern of the crystal form E, obtained using Cu-Kα radiation (λ=1.5406 Å) and expressed with diffraction angle 2*θ*, has characteristic peaks at 6.821, 10.035, 12.653, 13.727, and 14.787; preferably characteristic peaks at 6.821, 10.035, 12.653, 13.727, 14.787, 16.081, 16.648, 18.571, 20.151, and 22.204,
wherein the error range of the 2*θ* values is ± 0.2.

8. Crystal form F of a tromethamine salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein an X-ray powder diffraction pattern of the crystal form F, obtained using Cu-Kα radiation (λ=1.5406 Å) and expressed with diffraction angle 2*θ*, has characteristic peaks at 7.674, 10.614, 16.400, and 18.645; preferably characteristic peaks at 6.777, 7.674, 10.614, 11.594, 14.408, 14.882, 16.400, and 18.645; more preferably characteristic peaks at 6.777, 7.674, 10.614, 11.594, 13.671, 14.408, 14.882, 16.400, 18.645, 20.849, 21.384, 21.731, 22.108, 24.721, 26.169, and 29.192,
wherein the error range of the 2*θ* values is ± 0.2.

9. Crystal form α of a potassium salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[d]imidazole-6-carboxylic acid, wherein an X-ray powder diffraction pattern of the crystal form α, obtained using Cu-Kα radiation (λ=1.5406 Å) and expressed with diffraction angle 2*θ*, has characteristic peaks at 9.564, 11.515, 14.683, 19.607, and 20.391; preferably characteristic peaks at 9.564, 11.515, 14.683, 16.058, 18.859, 19.607, 20.391, 22.592, 23.320, and 25.176; more preferably characteristic peaks at 9.564, 11.515, 14.683, 16.058, 17.033, 18.859, 19.607, 20.391, 21.064, 22.592, 23.320, 24.449, 25.176, 25.933, 27.080, and 27.708,
wherein the error range of the 2*θ* values is ± 0.2.

10. Crystal form I of a sodium salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[d]imidazole-6-carboxylic acid,
wherein an X-ray powder diffraction pattern of the crystal form I, obtained using Cu-Kα radiation (λ=1.5406 Å) and expressed with diffraction angle 2*θ*, has characteristic peaks at 5.257, 5.806, 6.795, 10.106, 12.203, and 20.693,
wherein the error range of the 2*θ* values is ± 0.2.

11. Crystal form II of a sodium salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[d]imidazole-6-carboxylic acid,
wherein an X-ray powder diffraction pattern of the crystal form II, obtained using Cu-Kα radiation (λ=1.5406 Å) and expressed with diffraction angle 2*θ*, has characteristic peaks at 9.754, 11.731, and 19.730; more preferably characteristic peaks at 5.574, 9.754, 11.731, 14.856, 16.091, 19.730, and 22.670,
wherein the error range of the 2*θ* values is ± 0.2.

12. Crystal form I of a meglumine salt
of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern of the crystal form I, obtained using Cu-Kα radiation (λ=1.5406 Å) and expressed with diffraction angle 2*θ*, has characteristic peaks at 5.498, 11.013, 14.626, and 17.942; preferably characteristic peaks at 5.498, 8.901, 11.013, 14.626, 17.942, 19.454, 22.668, and 25.696; more preferably characteristic peaks at 5.498, 8.314, 8.901, 11.013, 11.891, 12.810, 14.626, 15.683, 15.931, 17.942, 18.748, 19.454, 20.393, 21.419, 22.234, 22.668, 23.391, 24.739, and 25.696,
wherein the error range of the 2*θ* values is ± 0.2.

13. Crystal form I of a p-toluenesulfonate salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein an X-ray powder diffraction pattern of the crystal form I, obtained using Cu-Kα radiation (λ=1.5406 Å) and expressed with diffraction angle 2*θ*, has characteristic peaks at 5.453, 5.884, 8.063, 12.925, 16.071, and 19.778; preferably characteristic peaks at 5.453, 5.884, 8.063, 12.925, 13.825, 15.399, 16.071, 18.231, 19.778, and 21.917; more preferably characteristic peaks at 5.453, 5.884, 8.063, 12.925, 13.825, 15.399, 16.071, 16.560, 17.066, 18.231, 19.778, 20.861, 21.917, 23.898, and 26.744,
wherein the error range of the 2*θ* values is ± 0.2.

14. Crystal form I of a tartrate salt of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[d]imidazole-6-carboxylic acid,
wherein an X-ray powder diffraction pattern of the crystal form I, obtained using Cu-Kα radiation (λ=1.5406 Å) and expressed with diffraction angle 2*θ*, has characteristic peaks at 12.810, 18.824, 21.890, and 24.472; preferably characteristic peaks at 12.810, 14.448, 15.604, 18.824, 20.410, 21.890, and 24.472; more preferably characteristic peaks at 9.342, 10.132, 10.858, 11.272, 12.810, 14.448, 15.604, 18.824, 20.410, 21.890, 24.472, and 27.880, wherein the error range of the 2*θ* values is ± 0.2.

15. Crystal form I of a hydrochloride salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid,
wherein an X-ray powder diffraction pattern of the crystal form I, obtained using Cu-Kα radiation (λ=1.5406 Å) and expressed with diffraction angle 2*θ*, has characteristic peaks at 9.939, 14.333, 14.933, 17.523, 18.480, and 20.134; preferably characteristic peaks at 9.939, 13.123, 13.649, 14.333, 14.933, 16.616, 17.523, 18.480, 19.378, 20.134, 20.988, 26.399, and 26.970; more preferably characteristic peaks at 9.939, 12.267, 13.123, 13.649, 14.333, 14.933, 16.616, 17.523, 18.480, 19.378, 20.134, 20.988, 22.377, 23.002, 24.477, 25.322, 26.399, 26.970, 27.609, 30.822, and 33.760,
wherein the error range of the 2*θ* values is ± 0.2.

16. Crystal form α of a malate salt of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[d]imidazole-6-carboxylic acid, wherein an X-ray powder diffraction pattern of the crystal form α, obtained using Cu-Kα radiation (λ=1.5406 Å) and expressed with diffraction angle 2*θ*, has characteristic peaks at 10.050, 14.508, 15.693, 18.924, and 20.240; preferably characteristic peaks at 10.050, 10.818, 11.213, 12.871, 14.508, 15.693, 18.924, 20.240, 21.829, and 24.396; more preferably characteristic peaks at 9.364, 10.050, 10.818, 11.213, 12.871, 13.774, 14.508, 15.693, 17.760, 18.924, 20.240, 21.829, 24.396, 26.200, 27.936, and 28.188, wherein the error range of the 2*θ* values is ± 0.2.

17. A pharmaceutical composition comprising the pharmaceutically acceptable salt according to claim 1 or 2 or the crystal form according to any one of claims 3-16, and optionally a pharmaceutically acceptable excipient.

18. The crystal form according to any one of claims 3-16 or the composition according to claim 17 for use in the treatment or prevention of diabetes.

## Patentansprüche

1. Pharmazeutisch verträgliches Salz der Verbindung 2-((4-((S)-3-(4-chloro-2-fluorphenyl)-2,3-dihydrobenzo[*b*][1,4]dioxan-5-yl)piperidin-1-yl)methyl)-1-(((S)-oxetan-2-yl)methyl)-1H-benzo[d]imidazol-6-Carbonsäure, wobei das pharmazeutisch verträgliche Salz ausgewählt ist aus einem Tromethaminsalz, einem Ammoniumsalz, einem Kaliumsalz, einem Argininsalz, einem Natriumsalz, einem Megluminsalz, einem Ethanolaminsalz, einem p-Toluolsulfonatsalz, einem Tartratsalz, einem Sulfatsalz, einem Malatsalz und einem Hydrochloridsalz.

2. Pharmazeutisch verträgliches Salz nach Anspruch 1, wobei das chemische Verhältnis der Verbindung 2-((4-((S)-3-(4-chloro-2-fluorphenyl)-2,3-dihydrobenzo[*b*][1,4]dioxan-5-yl)piperidin-1-yl)methyl)-1-(((S)-oxetan-2-yl)methyl)-1H-benzo[d]imidazol-6-Carbonsäure zu einem Alkalimolekül oder einem Säuremolekül 1:0,5 bis 1:3, bevorzugt 1:0,5, 1:1, 1:2 oder 1:3, am meisten bevorzugt 1:1 oder 1:2, ist.

3. Kristallform A eines Tromethaminsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform A, das mithilfe von Cu-Kα-Strahlung (λ = 1,5406 Ä) erhalten und mit einem Beugungswinkel 2*θ* ausgedrückt wird, charakteristische Peaks bei 6,850, 9,982, 13,789, 16,148 und 22,138; bevorzugt charakteristische Peaks bei 6,850, 9,982, 12,697, 13,789, 15,763, 16,148, 18,016, 19,016 und 22,138; mehr bevorzugt charakteristische Peaks bei 6,850, 8,051, 9,982, 12,697, 13,789, 14,704, 14,990, 15,763, 16,148, 18,016, 19,016, 20,109, 22,138, 25,712 und 27,909, aufweist,
wobei der Fehlerbereich der 2*θ*-Werte ± 0,2 ist.

4. Kristallform B eines Tromethaminsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform B, das mithilfe von Cu-Kα-Strahlung (λ = 1,5406 Ä) erhalten und mit dem Beugungswinkel 2*θ* ausgedrückt wird, charakteristische Peaks bei 8,144, 10,511, 12,290, 20,632 und 21,699; bevorzugt charakteristische Peaks bei 8,144, 10,511, 12,290, 13,996, 14,665, 20,632, 21,699 und 24,743; mehr bevorzugt charakteristische Peaks bei 8,144, 10,511, 12,290, 13,996, 14,665, 15,607, 16,520, 18,883, 20,632, 21,699, 23,741, 24,743, 26,055 und 27,035, aufweist,
wobei der Fehlerbereich der 2*θ*-Werte ± 0,2 ist.

5. Kristallform C eines Tromethaminsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform C, das mithilfe von Cu-Kα-Strahlung (λ = 1,5406 Ä) erhalten und mit dem Beugungswinkel 2*θ* ausgedrückt wird, charakteristische Peaks bei 6,551, 9,269, 13,175 und 16,906; bevorzugt charakteristische Peaks bei 6,551, 9,269, 13,175, 14,802, 15,864, 16,906, 18,776, 20,430 und 25,496; mehr bevorzugt charakteristische Peaks bei 6,551, 8,392, 9,269, 13,175, 14,802, 15,864, 16,906, 18,776, 20,430, 22,211, 22,922, 23,574, 25,496 und 26,290, aufweist, wobei der Fehlerbereich der 2*θ*-Werte ± 0,2 ist.

6. Kristallform D eines Tromethaminsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform D, das mithilfe von Cu-Kα-Strahlung (λ = 1,5406 Ä) erhalten und mit dem Beugungswinkel 2*θ* ausgedrückt wird, charakteristische Peaks bei 5,281, 10,292, 13,322 und 21,390; bevorzugt charakteristische Peaks bei 5,281, 9,912, 10,292, 10,961, 13,322, 21,390, 22,215, 23,979, 25,029 und 25,846; mehr bevorzugt charakteristische Peaks bei 5,281, 9,912, 10,292, 10,961, 11,613, 13,322, 15,351, 18,283, 19,214, 21,390, 22,215, 23,471, 23,979, 25,029, 25,846, 27,918 und 30,121, aufweist,
wobei der Fehlerbereich der 2*θ*-Werte ± 0,2 ist.

7. Kristallform E eines Tromethaminsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform E, das mithilfe von Cu-Kα-Strahlung (λ = 1,5406 Ä) erhalten und mit dem Beugungswinkel 2*θ* ausgedrückt wird, charakteristische Peaks bei 6,821, 10,035, 12,653, 13,727 und 14,787; bevorzugt charakteristische Peaks bei 6,821, 10,035, 12,653, 13,727, 14,787, 16,081, 16,648, 18,571, 20,151 und 22,204, aufweist, wobei der Fehlerbereich der 2*θ*-Werte ± 0,2 ist.

8. Kristallform F eines Tromethaminsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform F, das mithilfe von Cu-Kα-Strahlung (λ = 1,5406 Ä) erhalten und mit dem Beugungswinkel 2*θ* ausgedrückt wird, charakteristische Peaks bei 7,674, 10,614, 16,400 und 18,645; bevorzugt charakteristische Peaks bei 6,777, 7,674, 10,614, 11,594, 14,408, 14,882, 16,400 und 18,645; mehr bevorzugt charakteristische Peaks bei 6,777, 7,674, 10,614, 11,594, 13,671, 14,408, 14,882, 16,400, 18,645, 20,849, 21,384, 21,731, 22,108, 24,721, 26,169 und 29,192, aufweist,
wobei der Fehlerbereich der 2*θ*-Werte ± 0,2 ist.

9. Kristallform α eines Kaliumsalzes der Verbindung 2-((4-((S)-3-(4-chloro-2-fluorphenyl)-2,3-dihydrobenzo[*b*][1,4]dioxan-5-yl)piperidin-1-yl)methyl)-1-(((S)-oxetan-2-yl)methyl)-1H-benzo[d]imidazol-6-Carbonsäure, wobei ein Röntgenpulverbeugungsmuster der Kristallform α, das mithilfe von Cu-Kα-Strahlung (λ = 1,5406 Å) erhalten und mit dem Beugungswinkel 2*θ* ausgedrückt wird, charakteristische Peaks bei 9,564, 11,515, 14,683, 19,607 und 20,391; bevorzugt charakteristische Peaks bei 9,564, 11,515, 14,683, 16,058, 18,859, 19,607, 20,391, 22,592, 23,320 und 25,176; mehr bevorzugt charakteristische Peaks bei 9,564, 11,515, 14,683, 16,058, 17,033, 18,859, 19,607, 20,391, 21,064, 22,592, 23,320, 24,449, 25,176, 25,933, 27,080 und 27,708, aufweist,
wobei der Fehlerbereich der 2*θ*-Werte ± 0,2 ist.

10. Kristallform I eines Natriumsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform I, das mithilfe von Cu-Kα-Strahlung (λ = 1,5406 Ä) erhalten und mit dem Beugungswinkel 2*θ* ausgedrückt wird, charakteristische Peaks bei 5,257, 5,806, 6,795, 10,106, 12,203 und 20,693 aufweist, wobei der Fehlerbereich der 2*θ*-Werte ± 0,2 ist.

11. Kristallform II eines Natriumsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform II, das mithilfe von Cu-Kα-Strahlung (λ = 1,5406 Ä) erhalten und mit dem Beugungswinkel 2*θ* ausgedrückt wird, charakteristische Peaks bei 9,754, 11,731 und 19,730; mehr bevorzugt charakteristische Peaks bei 5,574, 9,754, 11,731, 14,856, 16,091, 19,730 und 22,670, aufweist,
wobei der Fehlerbereich der 2*θ*-Werte ± 0,2 ist.

12. Kristallform I eines Megluminsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform I, das mithilfe von Cu-Kα-Strahlung (λ = 1,5406 Ä) erhalten und mit dem Beugungswinkel 2*θ* ausgedrückt wird, charakteristische Peaks bei 5,498, 11,013, 14,626 und 17,942 aufweist; bevorzugt charakteristische Peaks bei 5,498, 8,901, 11,013, 14,626, 17,942, 19,454, 22,668 und 25,696; mehr bevorzugt charakteristische Peaks bei 5,498, 8,314, 8,901, 11,013, 11,891, 12,810, 14,626, 15,683, 15,931, 17,942, 18,748, 19,454, 20,393, 21,419, 22,234, 22,668, 23,391, 24,739 und 25,696,
wobei der Fehlerbereich der 2*θ*-Werte ± 0,2 ist.

13. Kristallform I eines p-Toluolsulfonatsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform I, das mithilfe von Cu-Kα-Strahlung (λ = 1,5406 Å) erhalten und mit dem Beugungswinkel 2*θ* ausgedrückt wird, charakteristische Peaks bei 5,453, 5,884, 8,063, 12,925, 16,071 und 19,778; bevorzugt charakteristische Peaks bei 5,453, 5,884, 8,063, 12,925, 13,825, 15,399, 16,071, 18,231, 19,778 und 21,917; mehr bevorzugt charakteristische Peaks bei 5,453, 5,884, 8,063, 12,925, 13,825, 15,399, 16,071, 16,560, 17,066, 18,231, 19,778, 20,861, 21,917, 23,898 und 26,744, aufweist,
wobei der Fehlerbereich der 2*θ*-Werte ± 0,2 ist.

14. Kristallform I eines Tartratsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform I, das mithilfe von Cu-Kα-Strahlung (λ = 1,5406 Å) erhalten und mit dem Beugungswinkel 2*θ* ausgedrückt wird, charakteristische Peaks bei 12,810, 18,824, 21,890 und 24,472; bevorzugt charakteristische Peaks bei 12,810, 14,448, 15,604, 18,824, 20,410, 21,890 und 24,472; mehr bevorzugt charakteristische Peaks bei 9,342, 10,132, 10,858, 11,272, 12,810, 14,448, 15,604, 18,824, 20,410, 21,890, 24,472 und 27,880, aufweist,
wobei der Fehlerbereich der 2*θ*-Werte ± 0,2 ist.

15. Kristallform I eines Hydrochloridsalzes der Verbindung 2-((4-((*S*)-3-(4-chloro-2-fluorphenyl)-2,3-dihydrobenzo[b][1,4]dioxan-5-yl)piperidin-1-yl)methyl)-1-(((*S*)-oxetan-2-yl)methyl)-1*H*-benzo[*d*]imidazol-6-Carbonsäure, wobei ein Röntgenpulverbeugungsmuster der Kristallform I, das mithilfe von Cu-Kα-Strahlung (λ = 1,5406 Ä) erhalten und mit dem Beugungswinkel 2*θ* ausgedrückt wird, charakteristische Peaks bei 9,939, 14,333, 14,933, 17,523, 18,480 und 20,134; bevorzugt charakteristische Peaks bei 9,939, 13,123, 13,649, 14,333, 14,933, 16,616, 17,523, 18,480, 19,378, 20,134, 20,988, 26,399 und 26,970; mehr bevorzugt charakteristische Peaks bei 9,939, 12,267, 13,123, 13,649, 14,333, 14,933, 16,616, 17,523, 18,480, 19,378, 20,134, 20,988, 22,377, 23,002, 24,477, 25,322, 26,399, 26,970, 27,609, 30,822 und 33,760, aufweist,
wobei der Fehlerbereich der 2*θ*-Werte ± 0,2 ist.

16. Kristallform α eines Malatsalzes der Verbindung 2-((4-((S)-3-(4-chloro-2-fluorphenyl)-2,3-dihydrobenzo[*b*][1,4]dioxan-5-yl)piperidin-1-yl)methyl)-1-(((S)-oxetan-2-yl)methyl)-1H-benzo[d]imidazol-6-Carbonsäure, wobei ein Röntgenpulverbeugungsmuster der Kristallform α, das mithilfe von Cu-Kα-Strahlung (λ = 1,5406 Å) erhalten und mit dem Beugungswinkel 2*θ* ausgedrückt wird, charakteristische Peaks bei 10,050, 14,508, 15,693, 18,924 und 20,240; bevorzugt charakteristische Peaks bei 10,050, 10,818, 11,213, 12,871, 14,508, 15,693, 18,924, 20,240, 21,829 und 24,396; mehr bevorzugt charakteristische Peaks bei 9,364, 10,050, 10,818, 11,213, 12,871, 13,774, 14,508, 15,693, 17,760, 18,924, 20,240, 21,829, 24,396, 26,200, 27,936 und 28,188, aufweist,
wobei der Fehlerbereich der 2*θ*-Werte ± 0,2 ist.

17. Pharmazeutische Zusammensetzung, umfassend das pharmazeutisch verträgliche Salz nach Anspruch 1 oder 2 oder die Kristallform nach einem der Ansprüche 3 bis 16 und optional einen pharmazeutisch verträglichen Hilfsstoff.

18. Kristallform nach einem der Ansprüche 3 bis 16 oder Zusammensetzung nach Anspruch 17 zur Verwendung bei der Behandlung oder Vorbeugung von Diabetes.

## Revendications

1. Sel pharmaceutiquement acceptable du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans lequel le sel pharmaceutiquement acceptable est choisi parmi un sel de trométhamine, un sel d'ammonium, un sel de potassium, un sel d'arginine, un sel de sodium, un sel de méglumine, un sel d'éthanolamine, un sel de p-toluènesulfonate, un sel de tartrate, un sel de sulfate, un sel de malate et un sel de chlorhydrate.

2. Sel pharmaceutiquement acceptable selon la revendication 1, dans lequel le rapport chimique du composé acide 2-((4-((S)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H-*benzo[d]imidazole-6-carboxylique à une molécule alcaline ou à une molécule acide est de 1:0,5 à 1:3, de préférence 1:0,5, 1:1, 1:2 ou 1:3, de manière préférée entre toutes 1:1 ou 1:2.

3. Forme cristalline A d'un sel de trométhamine du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline A, obtenu en utilisant un rayonnement Cu-Kα (λ=1,5406 Å) et exprimé avec un angle de diffraction 2*θ*, présente des pics caractéristiques à 6,850, 9,982, 13,789, 16,148 et 22,138 ; de préférence des pics caractéristiques à 6,850, 9,982, 12,697, 13,789, 15,763, 16,148, 18,016, 19,016 et 22,138 ; plus préférablement des pics caractéristiques à 6,850, 8,051, 9,982, 12,697, 13,789, 14,704, 14,990, 15,763, 16,148, 18,016, 19,016, 20,109, 22,138, 25,712 et 27,909, dans laquelle la plage d'erreur des valeurs 2*θ* est de ± 0,2.

4. Forme cristalline B d'un sel de trométhamine du composé acide 2-((4-((*S*)-3-(4-chloro-2-fluorophényl)-2,3-dihydrobenzo[b][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline B, obtenu en utilisant un rayonnement Cu-Kα (λ=1,5406 Å) et exprimé avec un angle de diffraction 2*θ*, présente des pics caractéristiques à 8,144, 10,511, 12,290, 20,632 et 21,699 ; de préférence des pics caractéristiques à 8,144, 10,511, 12,290, 13,996, 14,665, 20,632, 21,699 et 24,743 ; plus préférablement des pics caractéristiques à 8,144, 10,511, 12,290, 13,996, 14,665, 15,607, 16,520, 18,883, 20,632, 21,699, 23,741, 24,743, 26,055 et 27,035,
dans laquelle la plage d'erreur des valeurs 2*θ* est de ± 0,2.

5. Forme cristalline C d'un sel de trométhamine du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline C, obtenu en utilisant un rayonnement Cu-Kα (λ=1,5406 Å) et exprimé avec un angle de diffraction 2*θ*, présente des pics caractéristiques à 6,551, 9,269, 13,175 et 16,906 ; de préférence des pics caractéristiques à 6,551, 9,269, 13,175, 14,802, 15,864, 16,906, 18,776, 20,430 et 25,496 ; plus préférablement des pics caractéristiques à 6,551, 8,392, 9,269, 13,175, 14,802, 15,864, 16,906, 18,776, 20,430, 22,211, 22,922, 23,574, 25,496 et 26,290,
dans laquelle la plage d'erreur des valeurs 2*θ* est de ± 0,2.

6. Forme cristalline D d'un sel de trométhamine du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle (4) un diagramme de diffraction des rayons X sur poudre de la forme cristalline D, obtenu en utilisant un rayonnement Cu-Kα (λ=1,5406 Å) et exprimé avec un angle de diffraction 2*θ*, présente des pics caractéristiques à 5,281, 10,292, 13,322 et 21,390 ; de préférence des pics caractéristiques à 5,281, 9,912, 10,292, 10,961, 13,322, 21,390, 22,215, 23,979, 25,029 et 25,846 ; plus préférablement des pics caractéristiques à 5,281, 9,912, 10,292, 10,961, 11,613, 13,322, 15,351, 18,283, 19,214, 21,390, 22,215, 23,471, 23,979, 25,029, 25,846, 27,918 et 30,121,
dans laquelle la plage d'erreur des valeurs 2*θ* est de ± 0,2.

7. Forme cristalline E d'un sel de trométhamine du composé acide 2-((4-((*S*)-3-(4-chloro-2-fluorophényl)-2,3-dihydrobenzo[b][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline E, obtenu en utilisant un rayonnement Cu-Kα (λ=1,5406 Å) et exprimé avec un angle de diffraction 2*θ*, présente des pics caractéristiques à 6,821, 10,035, 12,653, 13,727 et 14,787 ; de préférence des pics caractéristiques à 6,821, 10,035, 12,653, 13,727, 14,787, 16,081, 16,648, 18,571, 20,151 et 22,204,
dans laquelle la plage d'erreur des valeurs 2*θ* est de ± 0,2.

8. Forme cristalline F d'un sel de trométhamine du composé acide 2-((4-((*S*)-3-(4-chloro-2-fluorophényl)-2,3-dihydrobenzo[b][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline F, obtenu en utilisant un rayonnement Cu-Kα (λ=1,5406 Å) et exprimé avec un angle de diffraction 2*θ*, présente des pics caractéristiques à 7,674, 10,614, 16,400 et 18,645 ; de préférence des pics caractéristiques à 6,777, 7,674, 10,614, 11,594, 14,408, 14,882, 16,400 et 18,645 ; plus préférablement des pics caractéristiques à 6,777, 7,674, 10,614, 11,594, 13,671, 14,408, 14,882, 16,400, 18,645, 20,849, 21,384, 21,731, 22,108, 24,721, 26,169 et 29,192,
dans laquelle la plage d'erreur des valeurs 2*θ* est de ± 0,2.

9. Forme cristalline α d'un sel de potassium du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline α, obtenu en utilisant un rayonnement Cu-Kα (λ=1,5406 Å) et exprimé avec un angle de diffraction 2*θ*, présente des pics caractéristiques à 9,564, 11,515, 14,683, 19,607 et 20,391 ; de préférence des pics caractéristiques à 9,564, 11,515, 14,683, 16,058, 18,859, 19,607, 20,391, 22,592, 23,320 et 25,176 ; plus préférablement des pics caractéristiques à 9,564, 11,515, 14,683, 16,058, 17,033, 18,859, 19,607, 20,391, 21,064, 22,592, 23,320, 24,449, 25,176, 25,933, 27,080 et 27,708, dans laquelle la plage d'erreur des valeurs 2*θ* est de ± 0,2.

10. Forme cristalline I d'un sel de sodium du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline I, obtenu en utilisant un rayonnement Cu-Kα (λ=1,5406 Å) et exprimé avec un angle de diffraction 2*θ*, présente des pics caractéristiques à 5,257, 5,806, 6,795, 10,106, 12,203 et 20,693,
dans laquelle la plage d'erreur des valeurs 2*θ* est de ± 0,2.

11. Forme cristalline II d'un sel de sodium du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline II, obtenu en utilisant un rayonnement Cu-Kα (λ=1,5406 Å) et exprimé avec un angle de diffraction 2*θ*, présente des pics caractéristiques à 9,754, 11,731 et 19,730 ; plus préférablement des pics caractéristiques à 5,574, 9,754, 11,731, 14,856, 16,091, 19,730 et 22,670,
dans laquelle la plage d'erreur des valeurs 2*θ* est de ± 0,2.

12. Forme cristalline I d'un sel de méglumine du composé acide 2-((4-((*S*)-3-(4-chloro-2-fluorophényl)-2,3-dihydrobenzo[b][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline I, obtenu en utilisant un rayonnement Cu-Kα (λ=1,5406 Å) et exprimé avec un angle de diffraction 2*θ*, présente des pics caractéristiques à 5,498, 11,013, 14,626 et 17,942 ; de préférence des pics caractéristiques à 5,498, 8,901, 11,013, 14,626, 17,942, 19,454, 22,668 et 25,696 ; plus préférablement des pics caractéristiques à 5,498, 8,314, 8,901, 11,013, 11,891, 12,810, 14,626, 15,683, 15,931, 17,942, 18,748, 19,454, 20,393, 21,419, 22,234, 22,668, 23,391, 24,739 et 25,696, dans laquelle la plage d'erreur des valeurs 2*θ* est de ± 0,2.

13. Forme cristalline I d'un sel de p-toluènesulfonate du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline I, obtenu en utilisant un rayonnement Cu-Kα (λ=1,5406 Å) et exprimé avec un angle de diffraction 2*θ*, présente des pics caractéristiques à 5,453, 5,884, 8,063, 12,925, 16,071 et 19,778 ; de préférence des pics caractéristiques à 5,453, 5,884, 8,063, 12,925, 13,825, 15,399, 16,071, 18,231, 19,778 et 21,917 ; plus préférablement des pics caractéristiques à 5,453, 5,884, 8,063, 12,925, 13,825, 15,399, 16,071, 16,560, 17,066, 18,231, 19,778, 20,861, 21,917, 23,898 et 26,744, dans laquelle la plage d'erreur des valeurs 2*θ* est de ± 0,2.

14. Forme cristalline I d'un sel de tartrate du composé acide 2-((4-((*S*)-3-(4-chloro-2-fluorophényl)-2,3-dihydrobenzo[b][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline I, obtenu en utilisant un rayonnement Cu-Kα (λ=1,5406 Å) et exprimé avec un angle de diffraction 2*θ*, présente des pics caractéristiques à 12,810, 18,824, 21,890 et 24,472 ; de préférence des pics caractéristiques à 12,810, 14,448, 15,604, 18,824, 20,410, 21,890 et 24,472 ; plus préférablement des pics caractéristiques à 9,342, 10,132, 10,858, 11,272, 12,810, 14,448, 15,604, 18,824, 20,410, 21,890, 24,472 et 27,880,
dans laquelle la plage d'erreur des valeurs 2*θ* est de ± 0,2.

15. Forme cristalline I d'un sel de chlorhydrate du composé acide 2-((4-((*S*)-3-(4-chloro-2-fluorophényl)-2,3-dihydrobenzo[*b*][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline I, obtenu en utilisant un rayonnement Cu-Kα (λ=1,5406 Å) et exprimé avec un angle de diffraction 2*θ*, présente des pics caractéristiques à 9,939, 14,333, 14,933, 17,523, 18,480 et 20,134 ; de préférence des pics caractéristiques à 9,939, 13,123, 13,649, 14,333, 14,933, 16,616, 17,523, 18,480, 19,378, 20,134, 20,988, 26,399 et 26,970 ; plus préférablement des pics caractéristiques à 9,939, 12,267, 13,123, 13,649, 14,333, 14,933, 16,616, 17,523, 18,480, 19,378, 20,134, 20,988, 22,377, 23,002, 24,477, 25,322, 26,399, 26,970, 27,609, 30,822 et 33,760,
dans laquelle la plage d'erreur des valeurs 2*θ* est de ± 0,2.

16. Forme cristalline α d'un sel de malate du composé acide 2-((4-((*S*)-3-(4-chloro-2-fluorophényl)-2,3-dihydrobenzo[b][1,4]dioxan-5-yl)pipéridin-1-yl)méthyl)-1-(((*S*)-oxétan-2-yl)méthyl)-1*H*-benzo[*d*]imidazole-6-carboxylique, dans laquelle un diagramme de diffraction des rayons X sur poudre de la forme cristalline α, obtenu en utilisant un rayonnement Cu-Kα (λ=1,5406 Å) et exprimé avec un angle de diffraction 2*θ*, présente des pics caractéristiques à 10,050, 14,508, 15,693, 18,924 et 20,240 ; de préférence des pics caractéristiques à 10,050, 10,818, 11,213, 12,871, 14,508, 15,693, 18,924, 20,240, 21,829 et 24,396 ; plus préférablement des pics caractéristiques à 9,364, 10,050, 10,818, 11,213, 12,871, 13,774, 14,508, 15,693, 17,760, 18,924, 20,240, 21,829, 24,396, 26,200, 27,936 et 28,188, dans laquelle la plage d'erreur des valeurs 2*θ* est de ± 0,2.

17. Composition pharmaceutique comprenant le sel pharmaceutiquement acceptable selon la revendication 1 ou 2 ou la forme cristalline selon l'une quelconque des revendications 3 à 16, et éventuellement un excipient pharmaceutiquement acceptable.

18. Forme cristalline selon l'une quelconque des revendications 3 à 16 ou composition selon la revendication 17 pour une utilisation dans le traitement ou la prévention du diabète.
